# EUROPEAN PATENT SPECIFICATION

(11) **EP 4 601 713 B1**
(45) Date of publication and mention of the grant of the patent: **18.11.2026**
(21) Application number: 23789667.5
(22) Date of filing: 13.10.2023
(51) Int. Cl.: A61L 24/04

(54) **ADHESIVE COMPOSITIONS**
KLEBSTOFFZUSAMMENSETZUNGEN
COMPOSITIONS ADHÉSIVES

(30) Priority: 14.10.2022 GB 202215222
(43) Date of publication of application: 20.08.2025
(73) Proprietor: Lumina Adhesives AB, 41265 Göteborg (SE)
(72) Inventor: TUNIUS, Mats, 443-39 Lerum (SE); BOSAEUS, Niklas, 414-78 Göteborg (SE)
(74) Representative: Gill Jennings & Every LLP
(86) International application number: PCT/EP2023/078569
(87) International publication number: WO 2024/079353

(56) References cited:
- WO-A1-2021/170711
- US-A1- 2018 030 321

## Description

### INTRODUCTION

This invention relates to pressure sensitive adhesive compositions. The invention relates in particular to adhesive compositions having low cytotoxicity which are therefore suitable for medical applications in which the adhesive composition contacts the skin directly. The invention also relates to methods for producing the adhesive compositions and to articles comprising the adhesive compositions.

The adhesive compositions of the invention may be used with various substrates, but they are particularly useful for application to the skin in view of their low cytotoxicity.

The adhesive compositions of the invention may comprise curable moieties and therefore may be switchable from a tacky state to a substantially non-tacky state when curing of the curable moieties is initiated by irradiation with light of a suitable wavelength. The loss of tackiness is evidenced by a reduction in the peel strength of the switched adhesive as compared to a non-switched adhesive.

Switchable adhesive compositions are particularly suitable for medical products that are applied to the skin of a patient. Before switching the adhesive forms a strong and durable bond to the skin, After switching, in a low tack state, the medical product can easily be removed without pain or damage to the underlying tissue. This is particularly useful for patients with injuries or long-term medical conditions requiring repeated application of dressings, as well as for patients with delicate skin (e.g. infants and the elderly).

### BACKGROUND OF THE INVENTION

Many medical devices comprise a layer of a pressure sensitive adhesive to enable the devices to be attached to the skin of a patient. Examples of such medical devices include dressings, surgical drapes and medical tape. Adhesives used in medical devices must be sufficiently tacky to form a strong bond to the skin that is able to resist peeling or accidental removal while the medical product is in use.

However, conventional adhesives having these properties can cause trauma and/or pain to the patient when the dressing or bandage is removed from the skin. This is particularly true for patients with long-term conditions that require an adhesive dressing to be applied to the same part of the body repeatedly over a prolonged period, such as stoma patients. It is also true for patients with delicate skin, particularly the elderly and infants.

A class of "switchable" pressure sensitive adhesives has been developed to address this problem. These adhesives have high initial peel strength but can be made to undergo physical and/or chemical changes that substantially reduce the peel strength before removal of an adhered product. The medical products can therefore be removed easily and without causing localised trauma or pain to a patient. To have practical utility for adhesive medical products it is furthermore necessary that a reduction in peel strength is achieved in a controlled manner in a relatively short time, say from a number of seconds to at most a few minutes. Furthermore, the adhesive itself and the switching mechanism must be suitable for use in contact with the skin.

For convenience, the term "switchable" is used herein to refer to adhesive compositions that can be changed from a tacky to a substantially non-tacky state. Typically, the reduction in peel force with the adhesives of the invention is around 99% on polished stainless steel and around 90% on skin.

One form of switchable adhesive is disclosed in US 5,032,637, US 5,352,516, US 4,331,576 and US 5,182,323. These documents describe adhesives that become less tacky upon contact with water. However, such adhesives are unsuitable for many medical applications where a patient's skin needs to be kept dry, for instance at a wound site.

US 2013/0123678, WO 2010/129299 and WO 2013/066401 disclose multilayer adhesive laminates that are selectively releasable from a substrate upon application of a liquid release agent to an outer face or perimeter of the laminate. The laminate comprises an interior layer and a plurality of fluid passageway conduits or apertures extending through the interior layer, an adhesive layer disposed along the bottom surface of the interior layer and a carrier layer disposed on the top surface of the interior layer. Removal of the carrier layer and application of an effective amount of the liquid release agent results in a loss of tackiness.

EP 0863775, US 6,184,264 and US 6,610,762 disclose adhesives that are switchable when exposed to visible light or low intensity UV light. The switchable adhesives described in these documents generally comprise an acrylic adhesive based on copolymers of alkyl acrylates, acrylic acid and/or a free radical polymerizable vinyl moiety "modified" or functionalised by a curable moiety bound thereto. Typical of the bound-in curable moieties are those derived from anthracenes, cinnamates, maleimides, coumarins, acrylates and/or methacrylates.

WO 2016/124339 and WO 2021/170711 by the current inventors disclose switchable adhesive compositions based on polyurethane adhesives. Unsaturated curable molecules may be mixed in and/or bound to the polyurethane polymer backbone. Photoinitiated curing of the curable molecules results in the formation of a crosslinked network which results in a reduction in peel strength of the adhesive.

Despite the developments described above, there remains a need in the art for improved switchable adhesives, which exhibit the properties of high peel strength before switching, low peel strength after switching, and low switching time once switching is initiated. It is found that adhesive formulations with good adhesive properties often demonstrate poor switching capability, and adhesive formulations with good switching capability often demonstrate low peel strength and/or low cohesiveness (the adhesive has low internal strength such that an adhered material will detach from a surface leaving residues of the adhesive on the surface). Obtaining high peel strength, high cohesiveness and good switching capability simultaneously is therefore a particular challenge when developing switchable adhesives.

There is furthermore a need for improved switchable adhesives in which the desirable adhesive and switching properties are obtained with low cytotoxicity.

Low cytotoxicity is of particular importance when the adhesive is used for medical applications where the adhesive may remain in contact with the skin for long periods of time. Adhesives that have cytotoxicity above a certain level can cause adverse skin effects, such as irritation and inflammation. Achieving low cytotoxicity and therefore good biocompatibility is one of the most pressing challenges in the development of adhesives for medical applications.

WO 2015/132551 by the present inventors also discloses switchable adhesive compositions based on polyurethane adhesives and having reduced cytotoxicity. Specifically, this document identifies low molecular weight compounds (e.g. having molecular weight in the range 150 to 450) as a source of cytotoxicity due to the ability of these compounds to penetrate the cell membrane as well as the skin.

There is an ongoing need in the art for switchable adhesive compositions with reduced cytotoxicity.

### SUMMARY OF THE INVENTION

The invention has been devised in view of the disadvantages of known switchable pressure sensitive adhesive systems as set out above and provides an improved switchable adhesive composition having high peel strength, high cohesiveness, fast switching times, a low switched peel strength, and low cytotoxicity.

In a first aspect, the invention provides an adhesive composition comprising the reaction product of:
(A) a polymer component having a weight average molecular weight in the range of 1,000 to 100,000 Dalton, and containing an average per molecule of X nucleophilic functional groups containing an active hydrogen atom, wherein X represents a number having a value of at least 2; and
(B) a cross-linking component obtained by reacting:
   (i) a polyisocyanate component having an average of 1.8 to 6 isocyanate functions per molecule;
   with at least one of:
   (ii) at least one compound comprising a functional group that is curable by free-radical polymerisation and further comprising a nucleophilic functional group containing an active hydrogen atom; and
   (iii) at least one compound comprising a nucleophilic functional group containing an active hydrogen atom and which does not include a functional group that is curable by free-radical polymerisation;
wherein the total degree of substitution of the polyisocyanate component (i) by the compounds (ii) and (iii) is in the range from 0.1 to 0.7; and wherein the molar ratio of unsubstituted isocyanate functions in the cross-linking component (B) to nucleophilic functional groups containing an active hydrogen atom in the polymer component (A) is at least 0.8.

In a second aspect, the invention provides a method of preparing an adhesive composition comprising:
(a) a first step of reacting:
   (i) a polyisocyanate component having an average of 1.8 to 6 isocyanate functions per molecule;

   with at least one of:
      (ii) at least one compound comprising a functional group that is curable by free-radical polymerisation and further comprising a nucleophilic functional group containing an active hydrogen atom; and
      (iii) at least one compound comprising a nucleophilic functional group containing an active hydrogen atom and which does not include a functional group that is curable by free-radical polymerisation;
   wherein the total degree of substitution of the polyisocyanate component (i) by the compounds (ii) and (iii) is in the range from 0.1 to 0.7; to form a cross-linking component (B);
(b) a second step of combining cross-linking component (B) formed in step (a) with a polymer component (A) having a weight average molecular weight in the range of 1,000 to 100,000 Dalton, and containing an average per molecule of X nucleophilic functional groups containing an active hydrogen atom, wherein X represents a number having a value of at least 2,
wherein the amounts of the cross-linking component (B) and the polyol component (A) are selected such that molar ratio of unsubstituted isocyanate functions in the cross-linking component (B) to nucleophilic functional groups containing an active hydrogen atom in the polymer component (A) is at least 0.8.

Also disclosed is an adhesive medical device comprising a layer of an adhesive composition as defined herein disposed on a first carrier film and a release liner disposed over the adhesive layer.

Wherein an adhesive medical product according to the third aspect is for use in a method of treating a wound, the method comprising removing the release liner and applying the adhesive dressing to the wound.

### DETAILED DESCRIPTION OF THE INVENTION

The adhesive composition of the invention is the product of a reaction between a polymer component (component (A)) and a cross-linking (component (B)). Specifically, the adhesive is formed by reaction of the nucleophilic functional groups on the polymer component with free isocyanate groups of the cross-linking component.

The cross-linking component (B) is obtained by partial reaction of a polyisocyanate component (i) with at least one of: compound (ii) that comprises a curable functional group; and compound (iii) which does not comprise a curable functional group. The total degree of substitution of the polyisocyanate component (i) by the compounds (ii) and (iii) is controlled within the specified ranges such that at least a portion of the isocyanate groups remain unreacted and therefore available for reaction with the hydroxy groups of the polymer component (A) to form a cross-linked adhesive network including curable groups.

The degree of substitution of the polyisocyanate component (i) represents the fraction of isocyanate groups of the polyisocyanate component (i) that are substituted by the nucleophilic functional groups containing an active hydrogen atom from compounds (ii) and (iii). It may be defined as follows: $degree of substitution = \frac{total moles nucleophilic groups in compound \left(ii\right) and compound \left(iii\right)}{total moles isocyanate groups in polyisocyanate component \left(i\right)}$

If compound (iii) is absent, then the total degree of substitution of the polyisocyanate component (i) relates to the degree of substitution by compound (ii). If compound (ii) is absent, then the total degree of substitution of the polyisocyanate component (i) relates to the degree of substitution by compound (iii). If both compounds (ii) and (iii) are present, then the total degree of substitution of the polyisocyanate component (i) relates to the total degree of substitution by compound (ii) and compound (iii).

As a result of the partial reaction of the polyisocyanate component (i) with the compound (ii) and/or the compound (iii), a product mixture is obtained that comprises a statistical mixture of substituted polyisocyanates. Some individual polyisocyanate molecules will be substituted by compound (ii) and/or (iii) at every isocyanate group, whereas other polyisocyanate molecules will not be substituted at all. Still other polyisocyanate molecules will include one or more substituted isocyanate groups and one or more unsubstituted isocyanate groups in the same molecule. The unsubstituted isocyanate groups are available for reaction with the nucleophilic functional groups of the polymer component (A).

In the case that an individual polyisocyanate molecule is fully substituted by compounds (ii) and/or (iii), the resulting compound will have no free isocyanate groups and will not be able to react with the polyol component (A). However, if that compound is substituted with compound (ii), it may function as an unbound curable molecule which is able to participate in the curing reaction to switch the adhesive from a high-tack to a low-tack state.

If an individual polyisocyanate molecule (i) is partially substituted by compounds (ii) and/or (iii), or is unsubstituted, it will be able to react with the nucleophilic functional groups of the polymer component and will thus become bound-in to the polyurethane adhesive via a urethane/urea/amide bond. Compounds in the cross-linking component (B) having only one unsubstituted isocyanate group will form a terminal bond to the polymer component and thus may function to bind curable molecules to the polymer backbone. Compounds in the cross-linking component (B) having two unsubstituted isocyanate groups can cross-link two nucleophilic groups of the polymer component to form a crosslinked polymeric network.

Compounds in the cross-linking component (B) having three (or more) unsubstituted isocyanate groups are able to crosslink three (or more) nucleophilic groups of the polymer component and thus form a nodal (or branching) point in the where multiple polymer end groups are bound to the same polyisocyanate molecule.

The degree of the substitution of the polyisocyanate component (i) by the compound (ii) and/or compound (iii) has an important effect on the properties of the adhesive composition. If the degree of substitution is too low, the polymer component (A) becomes too tightly bound into nodal points as defined above. As a consequence, the mobility of the adhesive is reduced, resulting in inadequate tackiness. On the other hand, if the degree of substitution is too high, the crosslinking component comprises relatively few molecules comprising two or more unsubstituted isocyanate groups that can crosslink the polymer component (A). With a low degree of crosslinking, the cohesiveness of the adhesive composition is poor and the adhesive is therefore difficult to apply and remove without leaving residues.

It has also been found that the tackiness of the adhesive composition depends on the ratio of isocyanate groups in the crosslinking component to nucleophilic groups in the polymer. This ratio is of prime importance in polyurethane chemistry and is often expressed as a percentage ([NCO]/[OH] × 100%). It has previously been considered that polyurethane adhesives for medical applications should have an NCO index of well below 100% since the additional mobility of the polymer chains adjacent the unbound hydroxy groups as well as the hydrogen-bonding ability of the unbound hydroxy groups contribute to the tackiness of the adhesive. For example, WO 2021/170711 teaches that the molar ratio of isocyanate functions in the crosslinking component to hydroxy groups in the polyol component is preferably from 0.5 to 0.7. WO 2015/075448 also relates to polyurethane medical adhesives and specifies a NCO index in the range from 0.45 to 0.69. EP2179749 discloses a pressure sensitive polyurethane adhesive for medical use and specifies a NCO index in the range from 0.03 to 0.5.

It has now been discovered that unbound nucleophilic groups and the adjacent polymer chains play an important role in the cytotoxicity of polyurethane adhesives. Efforts to reduce the cytotoxicity of polyurethane adhesives have so far focused on the presence of low molecular weight impurities and by-products with the ability to penetrate the skin and cell membranes. However, the present inventors have identified that the adhesive polymer itself can have a cytotoxic effect depending on its structure. It is believed that this cytotoxicity is due to the ability of unbound polymer chains to behave similarly to surfactants, with the ability to disrupt cell membranes.

It has now been found that a significant reduction in cytotoxicity is observed when the molar ratio of unsubstituted isocyanate functions in the cross-linking component (B) to nucleophilic (e.g. hydroxy) groups in the polymer component (A) is at least 0.8 (i.e. isocyanate index of at least 80%). A high isocyanate index ensures that a high proportion of polymer chains are bound by the cross-linking component (B) and are therefore unable to contribute to cytotoxicity as surfactants.

It has furthermore been found that sufficient tackiness (peel strength) is achieved when the cross-linking component is a polyisocyanate that is partially substituted by the compounds (ii) and/or (iii) as defined herein. Not only does the partial substitution of the polyisocyanate facilitate the incorporation of curable groups into the composition, but it also modifies the bonding of the polymer component to the polyisocyanate such that relatively few polymer chains are tightly bound into nodal points and the adhesive retains greater chain mobility, thereby contributing to increased tackiness. It is furthermore found that the statistical mixture of partially substituted isocyanate compounds obtained according to the invention provides an improved adhesive as compared to an adhesive that is formed using unsubstituted polyisocyanates having a lower number of isocyanate groups per molecule. Furthermore, less plasticisation and better cohesive strength is obtained when fewer unbound polymer molecules are present, since this leads to the cross-linking being more evenly distributed throughout the adhesive composition.

The adhesive compositions of the invention generally have a gel-like consistency with a relatively low density of cross-linking. The compositions are capable of forming polar bonds and Van der Waals bonds with substrates, and this give the adhesive composition its tackiness.

When the cross-linking component (B) contains curable moieties from compound (ii), a curing (or switching) reaction can be initiated, for example, by exposure to long wavelength UV or visible light in the presence of a photoinitiator. This curing reaction significantly increases the cross-linking density within the adhesive composition, compared to the unswitched adhesive composition. This reduces the mobility and free volume of the polymeric segments of the adhesive composition, meaning that the composition loses its flowability, essentially becoming an elastic film with no or little tackiness.

When a pressure sensitive adhesive is peeled from a surface to which it is adhered, the required energy may be about 10² to 10⁴ times larger than that which would be needed from a thermodynamic perspective, as a result of internal energy losses in the adhesive bulk material and because at least a component of the peeling force acts in the direction that is normal to the surface. In the switchable adhesive compositions of the invention, containing compound (ii) in the cross-linking component, the dense polymeric network that is formed by the curable functional groups upon switching of the adhesive reduces the tackiness and flexibility of the bulk material of the composition. The required peel force is therefore reduced to a value that is closer to that which is implied by thermodynamic considerations alone.

### Polymer component (A)

The polymer component has a weight average molecular weight in the range of 1,000 to 100,000 Dalton and contains an average per molecule of X nucleophilic functional groups containing an active hydrogen atom, wherein X represents a number having a value of at least 2. The nucleophilic groups of the polymer component (A) react with the free isocyanate groups of the cross-linking component (B) to form a cross-linked adhesive network.

The adhesive compositions of the invention are preferably polyurethane adhesives. Polyurethane adhesives are formed when the polymer component is a polyol, and thus wherein the nucleophilic groups are hydroxy groups. However, it is not excluded that the adhesive compositions of the invention may be formed from amine terminated polymers (which react with the isocyanate component to form a urea bond) or from carboxy-terminated polymers (which react with the isocyanate component to form an amide bond).

Preferred polyols comprise an average of 3 or more hydroxy groups per molecule, preferably 3 to 5 hydroxy groups per molecule.

Preferably, at least 50 mol% of the hydroxy groups in the polyol are found in polyol molecules comprising from 3 to 5 hydroxy groups, or wherein at least 60 mol% of the hydroxy groups in the polyol are found in polyol molecules comprising from 3 to 5 hydroxy groups, or wherein at least 70 mol% of the hydroxy groups in the polyol are found in polyol molecules comprising from 3 to 5 hydroxy groups, or wherein at least 80 mol% of the hydroxy groups in the polyol are found in polyol molecules comprising from 3 to 5 hydroxy groups, or wherein at least 90 mol% of the hydroxy groups in the polyol are found in polyol molecules comprising from 3 to 5 hydroxy groups.

More preferably, at least 50 mol% of the hydroxy groups in the polyol are found in polyol molecules comprising from 3 or 4 hydroxy groups, or wherein at least 60 mol% of the hydroxy groups in the polyol are found in polyol molecules comprising from 3 or 4 hydroxy groups, or wherein at least 70 mol% of the hydroxy groups in the polyol are found in polyol molecules comprising from 3 or 4 hydroxy groups, or wherein at least 80 mol% of the hydroxy groups in the polyol are found in polyol molecules comprising from 3 or 4 hydroxy groups, or wherein at least 90 mol% of the hydroxy groups in the polyol are found in polyol molecules comprising from 3 or 4 hydroxy groups.

More preferably, at least 50 mol% of the hydroxy groups in the polyol are found in polyol molecules comprising from 3 hydroxy groups, or wherein at least 60 mol% of the hydroxy groups in the polyol are found in polyol molecules comprising from 3 hydroxy groups, or wherein at least 70 mol% of the hydroxy groups in the polyol are found in polyol molecules comprising from 3 hydroxy groups, or wherein at least 80 mol% of the hydroxy groups in the polyol are found in polyol molecules comprising from 3 hydroxy groups, or wherein at least 90 mol% of the hydroxy groups in the polyol are found in polyol molecules comprising from 3 hydroxy groups.

Although diols are routinely used in the art to prepare polyurethane adhesives, it has been found that certain diols (including diols formed from polypropylene glycol end-capped with polyethylene glycol) have a larger effect on cytotoxicity than similar polyols comprising 3 or more hydroxy groups. Without being bound by theory it is believed that polyurethane moieties derived from diols retain some surfactant character even after reaction with the cross-linking component (B).

Meanwhile, it has also been found that adhesive compositions comprising a high proportion of polyols comprising 3 to 5 hydroxy groups retain sufficient tackiness even at the high molar ratio of unsubstituted isocyanate functions in the cross-linking component (B) to hydroxyl groups in the polyol component (A). This is particularly the case for adhesive compositions comprising a high proportion of polyols comprising 3 or 4 hydroxy groups, still more particularly for adhesive compositions comprising a high proportion of polyols comprising 3 hydroxy groups.

The use of polyols containing 3 or more hydroxy groups provides another source of crosslinking in the polyurethane structure. While this is beneficial to an extent, the use of polyols comprising very high numbers of hydroxy groups may result in excessive crosslinking and a loss of tackiness. Accordingly, it is preferred that the polyol component comprises primarily polyols comprising from 3 to 5 hydroxy groups, preferably 3 or 4 hydroxy groups, preferably 3 hydroxy groups.

The polymer component (A) is preferably selected from hydroxy-terminated polyethers (i.e. polyether polyols), and hydroxy-terminated polyesters (i.e. polyester polyols). Most preferably, the polymer component is a hydroxy-terminated polyether, more preferably a hydroxy-terminated polyether derived from ether units (monomers) comprising from 2 to 10 carbon atoms, preferably 2 to 6 carbon atoms, more preferably 2 to 4 carbon atoms. Particularly, preferred hydroxy-terminated polyethers comprise repeating units derived from ethylene oxide and/or propylene oxide.

Suitable hydroxy-terminated polyethers can be obtained by alkoxylation of starting molecules having the required number of nucleophilic groups. Preferred hydroxy-terminated polyethers include alkoxylated derivatives of compounds comprising from 3 to 5 hydroxy groups, or mixtures thereof. For example, suitable compounds comprising from 3 to 5 hydroxy groups that can be used as starter molecules for the formation of polyethers by alkoxylation include glycerol, trimethylolpropane, erythritol, pentaerythritol, pentane-1,2,4,5-tetrol, dextrose and mixtures thereof.

Preferred alkoxylated derivatives are ethoxylated derivates, propoxylated derivatives, or ethoxylated-co-propoxylated derivatives. Optionally, the hydroxy-terminated polyether may be an ethylene oxide capped propoxylated derivative of a compound comprising from 3 to 5 hydroxy groups, or a mixture thereof, including those described above. More preferably, the hydroxy-terminated polyether may be selected from an ethoxylated derivative of glycerol or trimethylolpropane, or an ethylene oxide capped propoxylated derivative of glycerol or trimethylolpropane. Ethylene oxide capping is preferred since low steric hindrance of the terminal hydroxy groups promotes faster reaction with the isocyanate groups of the cross-linking component (B).

The polymer component (A) preferably has a weight average molecular weight in the range of 1,000 to 50,000 Dalton, preferably in the range of 1,000 to 20,000 Dalton, preferably in the range of 1,500 to 10,000 Dalton (as measured by GPC).

The polymer component preferably has an equivalent weight per nucleophilic functional group containing an active hydrogen atom (per hydroxy group in the case that the polymer component is a polyol) of from 200 to 5,000, preferably from 500 to 2,500, preferably from 1,000 to 2,000.

X preferably represents a number having a value of at least 2.2, preferably at least 2.4, preferably at least 2.6, preferably at least 2.8, preferably at least 3, preferably at least 3.2. In particular, it is preferred that at least a portion of the polymer molecules in the polymer component (A) contain three or more nucleophilic functional groups. Polymer molecules having 3 or more nucleophilic functional groups with active hydrogen atoms are able to introduce further cross-linking into the polymeric network of the polyurethane adhesive which contributes to improved adhesive performance.

The value of X represents the nominal functionality of the polymer component. In the case that the polymer component is formed from a single starting molecule, the nominal functionality of the polymer is equivalent to the functionality of the starting molecule (e.g. nominal functionality of 3 if trimethylolpropane is used as a starting molecule). The nominal functionality may also be calculated as follows: $X = \frac{moles nucleophilic functional groups containing an active hydrogen atom in the polymer component \left(A\right)}{moles polymer molecules in polymer component \left(A\right)}$

In practice, this is equivalent to the number average molecular weight (by GPC) of the polymer divided by the equivalent weight per nucleophilic functional group containing an active hydrogen atom.

### Cross-linking component (B)

The cross-linking component (B) of the adhesive compositions of the invention is obtained by the reaction of a polyisocyanate component (i) with at least one of: compound (ii) that comprises a functional group that is curable by free-radical polymerisation and also comprises a nucleophilic functional group containing an active hydrogen atom; and/or compound (iii) that comprises a nucleophilic functional group containing an active hydrogen atom but that does not include a functional group that is curable by free-radical polymerisation.

If the polyisocyanate component (i) is reacted with the compound (ii), the functional group that is curable by free-radical polymerisation provides a switching capability to the adhesive composition. The polyisocyanate component (i) may optionally also be reacted with the compound (iii). Reaction of the polyisocyanate component (i) additionally with compound (iii) does not contribute to the switching capability of the adhesive, however it allows for further control over the degree of substitution of the polyisocyanate component (i) independently of the content of curable moieties in the adhesive.

If the polyisocyanate component (i) is reacted only with the compound (iii), and compound (ii) is omitted, the resulting adhesive is not switchable via a curing reaction. However, the polyurethane adhesive is nonetheless useful for medical applications due to the low cytotoxicity and the gentle behaviour of gel adhesives when peeled from skin achieved by the invention.

The total degree of substitution of the polyisocyanate component (i) by the compounds (ii) and/or (iii) is from 0.2 to 0.7. At very low degrees of substitution, the cross-linking component (B) comprises higher relative amounts of polyisocyanates that are either unsubstituted or mono-substituted. This results in tighter cross-linking of the polyol component, with more of the polymer end groups bound into nodal points with e.g. three or more polymer end groups attached. It has been found that this results in an adhesive having inadequate peel strength at the high isocyanate index required to obtain low cytotoxicity. Although effective adhesives may be obtained even with very high degrees of substitution of the polyisocyanate component (i), such compositions will require a higher total amount of the polyisocyanate to ensure sufficient free isocyanate groups are still available to cross-link the polyol component (A). This may be undesirable for cost reasons. Particularly good adhesive performance is found, for instance when the total degree of substitution of the polyisocyanate component (i) by the compounds (ii) and/or (iii), if present, is in the range from 0.25 to 0.65, preferably in the range from 0.3 to 0.6.

### Polyisocyanate component (i)

The polyisocyanate component (i) has an average of 1.8 to 6 isocyanate functions per molecule. Preferably the average number of isocyanate functions per molecule is in the range from 2 to 4, preferably in the range from 2.1 to 3.6, preferably in the range from 2.1 to 3.5, preferably in the range from 2.2 to 3.4, preferably in the range from 2.5 to 3.4. The term average is used herein to refer to the number based mean average as follows: $mean average of isocyanate functions = \frac{moles isocyanate functional groups in the polyisocyanate component \left(i\right)}{moles isocyanate - containing molecules in polyisocyanate component \left(i\right)}$

It will be understood that the polyisocyanate component (i) may be a single polyisocyanate compound or may comprise a mixture of different polyisocyanate compounds, so long as the average number of polyisocyanate groups per molecule is within the specified ranges.

The polyisocyanate component (i) may in principle be selected from any of the polyisocyanate compounds that are known in the art for the manufacture of polyisocyanates, as well as mixtures thereof. For example, the polyisocyanate component (i) may comprise one or more polyisocyanate compounds selected from the group of polyisocyanates containing from 2 to 10 isocyanate functions per molecule and mixtures thereof, so long as the average number of isocyanate functions per molecule is within the range from 1.8 to 6. Preferably, the polyisocyanate component (i) comprises one or more polyisocyanate compounds wherein the polyisocyanate component has an average of 2 to 4 isocyanate functions per molecule, preferably 2.1 to 3.6 isocyanate functions per molecule, preferably 2.1 to 3.5 isocyanate functions per molecule, preferably 2.2 to 3.4 isocyanate functions per molecule, preferably 2.5 to 3.4 isocyanate functions per molecule.

Optionally, the polyisocyanate component (i) may comprise a mixture of a diisocyanate and at least one polyisocyanate having an average of at least 3 isocyanate functions per molecule. It has been observed that mixtures of diisocyanates with other polyisocyanates result in adhesives which are more resistant to humidity. This is beneficial for medical applications in which the adhesive must stick to the skin, which may secrete sweat.

Examples of suitable polyisocyanates include diisocyanates of the formula OCN-R-NCO, wherein R independently represents a straight chain, branched or cyclic alkylene group having from 2 to 15 carbon atoms or an arylene group having from 6 to 20 carbon atoms. Examples of diisocyanates include hexamethylene diisocyanate, isophorone diisocyanate, toluene 2,4-diisocyanate, 4,4'-methylenebis(phenyl isocyanate), and 4,4'-methylenebis(cyclohexyl isocyanate). Further suitable polyisocyanates include polymers (e.g. dimers, trimers, tetramers etc.) based on the diisocyanates.

A preferred polyisocyanate is a trimerized diisocyanate of the formula D(R-NCO)₃, wherein D represents a ring structure selected from isocyanurate and iminooxadiazindione or a branched structure selected from biuret and allophanate and mixtures thereof, and each R independently represents a straight chain, branched or cyclic alkylene group having from 2 to 15 carbon atoms or an arylene group having from 6 to 20 carbon atoms. A particularly preferred polyisocyanate in this class is trimerized hexamethylene diisocyanate.

### Compound (ii)

The cross-linking component (B) may be obtained by the reaction of the polyisocyanate component (i) with at least one compound (ii) comprising a functional group that is curable by free-radical polymerisation and further comprises a nucleophilic functional group containing an active hydrogen atom.

The term "nucleophilic functional group containing an active hydrogen atom" refers herein to functional groups that are capable of undergoing an addition reaction with an isocyanate group to form an adduct. For example, the nucleophilic functional group containing an active hydrogen atom may be -OH, -COOH -NH, -SH, etc. Preferably the nucleophilic functional group containing an active hydrogen atom is a hydroxy group (-OH). Preferably, the nucleophilic functional group containing an active hydrogen atom is a hydroxy group attached to a primary carbon atom (i.e. a group of the formula -CH₂OH).

Nucleophilic hydroxy groups can readily react with the isocyanate groups of the polyisocyanate component (i) and hydroxy-containing compounds containing curable moieties are readily available in the art. Preferably, the at least one compound (ii) comprises a single nucleophilic functional group containing an active hydrogen atom.

The at least one compound (ii) preferably comprises an olefin moiety as the functional group that is curable by free-radical polymerisation. The at least one compound (ii) may be a single compound, or a mixture of different compounds that each comprise an olefin moiety as the functional group that is curable by free-radical polymerisation. Olefin moieties (e.g. acrylates and methacrylates) tend to have high yields in polymerisation reactions and are thus good curable groups for achieving effective switch performance via a free-radical initiated curing process.

Preferably, the at least one compound (ii) comprises an olefin moiety as the functional group that is curable by free-radical polymerisation and a hydroxy group as the nucleophilic functional group containing an active hydrogen atom.

In the broadest sense, any unsaturated compounds that are curable by free radical polymerization and further comprise a nucleophilic functional group containing an active hydrogen atom can be used as the compound (ii). Examples of suitable compounds include hydroxy-substituted acrylate esters and hydroxy-substituted methacrylate esters, and mixtures thereof. Preferably, the at least one compound (ii) is selected from hydroxy substituted-(C₂-C₂₀)alkyl methacrylate esters, hydroxy substituted-(C₂-C₂₀)alkyl acrylate esters, polyalkoxylated monomethacrylate esters containing 2-10 ether functions, polyalkoxylated monoacrylate esters containing 2-10 ether functions, and mixtures thereof. More preferably, the at least one compound (ii) is selected from hydroxy substituted-(C₂-C₆)alkyl methacrylate esters.

For example, the compound (ii) may be selected from 2-hydroxyethyl methacrylate, 2-hydroxypropyl methacrylate, 2-hydroxyethyl acrylate, 2-hydroxypropyl acrylate polypropylene glycol monomethacrylate, and polypropylene glycol monoacrylate. Preferred compounds (ii) are 2-hydroxypropyl methacrylate, 2-hydroxyethyl methacrylate and mixtures thereof.

The at least one compound (ii) may be a single compound, or may be a mixture of compounds, each of which has a functional group that is curable by free-radical polymerization and a nucleophilic functional group containing an active hydrogen atom. In the case that compound (ii) is a mixture of compounds, each compound preferably comprises the same type of curable functional group, more preferably each compound comprises an olefin group. For example, compound (ii) may be a mixture of 2-hydroxypropyl methacrylate with the isomeric hydroxyisopropyl methacrylate.

Other examples of hydroxy containing acrylic esters are hydroxy (CH₂)ₙ methacrylic esters where n is 4-8, hydroxyethyl methacrylate caprolactone ester (caprolactone 2-(methacryloyloxy)ethyl ester), 3-(acryloyloxy)-2-hydroxypropyl methacrylate and glycerol dimethacrylate.

The compound (ii) may optionally be used in combination with the compound (iii). As noted above, the total degree of substitution of the polyisocyanate component (i) by the compounds (ii) and (iii) is in the range from 0.1 to 0.7, and is preferably in the range from 0.15 to 0.65, more preferably in the range from 0.2 to 0.6, more preferably in the range from 0.25 to 0.6, more preferably in the range from 0.3 to 0.6.

The degree of substitution of the polyisocyanate component (i) by the compound (ii) is preferably in the range from 0.05 to 0.5; or from 0.05 to 0.45, or from 0.05 to 0.4, or from 0.8 to 0.35, or from 0.8 to 0.3, or from 0.1 to 0.25, or from 0.1 to 0.2, or from 0.12 to 0.18. The amount of compound (iii), if any, may then be selected to achieve a total degree of substitution as set out above. At very low degrees of substitution of the polyisocyanate component (i) with the compound (ii), there is a lower relative amount of curable groups which may result in reduced switch performance of the adhesive upon curing. However, much higher levels of curable groups exceed what is necessary for effective switching and therefore add to the cost of producing the adhesive without any substantial performance benefit.

### Compound (iii)

The cross-linking component (B) may be obtained by reacting the polyisocyanate component (i) with both the compound (ii) and the compound (iii).

As set out above, partial substitution of the polyisocyanate component (i) is desirable to obtain a distribution of different isocyanate-containing compounds in the cross-linking component (B). However, obtaining the required degree of substitution of the polyisocyanate component (i) with only the compound (ii) tends to result in an adhesive comprising significantly more curable groups than are necessary to obtain good switch performance. Excessive amounts of curable groups may necessitate careful handling during manufacture of the adhesive and the products that comprise the adhesive to avoid premature switching. Excessive substitution of the polyisocyanate component (i) by the compound (ii) is also disfavoured for cost and biocompatibility reasons.

One particular consequence of compositions comprising a high concentration of curable groups is that shear forces in the apparatus (e.g. pumps and flow lines) used to handle adhesives or their precursors during manufacturing processes may initiate premature curing of the curable groups. This may result in reduced tackiness of the adhesive product and in some cases cause blockage of the manufacturing apparatus. The use of the compound (iii) therefore allows these problems to be mitigated by enabling the required degree of substitution of the polyisocyanate component (i) without introducing excessive curable groups into the adhesive composition.

In an alternative embodiment, the cross-linking component (B) may be obtained by reacting the polyisocyanate component (i) with the compound (iii), wherein the compound (ii) is omitted. In this case, the compound (iii) alone provides for the partial substitution of the polyisocyanate component (i) in the absence of a curable component.

Where the compound (ii) is omitted, the total degree of substitution of the polyisocyanate component (i) is equivalent to the degree of substitution of the polyisocyanate component (i) by the compound (iii) and is therefore in the range from 0.2 to 0.7, and is preferably in the range from 0.25 to 0.65, more preferably in the range from 0.3 to 0.6, more preferably in the range from 0.35 to 0.6, more preferably in the range from 0.4 to 0.6.

The at least one compound (iii) comprises a nucleophilic functional group containing an active hydrogen atom but does not include a functional group that is curable by free radical polymerisation. The at least one compound (iii) may be a single compound, or it may be a mixture of different compounds, none of which includes a functional group that is curable by free-radical polymerisation.

The at least one compound (iii) is preferably selected from linear, branched or cyclic C₁-C₃₀ aliphatic alcohols, preferably linear, branched or cyclic C₁-C₁₈ aliphatic alcohols, more preferably linear, branched or cyclic C₁-C₁₂ aliphatic alcohols. Preferably the at least one compound (iii) is selected from branched C₃-C₁₂ aliphatic alcohols, or from branched C₆-C₁₈ aliphatic alcohols. An example of a suitable compound is 2-ethyl-1-hexanol. Compounds of this type occupy one or more isocyanate bonding sites so as to obtain the required distribution of reaction products from the reaction of the polyisocyanate component (i) with the compound (iii) and optionally compound (ii).

However, a further advantage of including the compounds (iii) is that the aliphatic carbon chain adds a degree of fattiness to the adhesive composition. This reduces the water solubility of the adhesive as a whole and in particular reduces the ability of any constituents of the adhesive having relatively low molecular weight to penetrate the skin. The compound (iii) therefore provides a further reduction in the cytotoxicity of the adhesive. Furthermore, the aliphatic carbon chains act as a plasticiser in the adhesive, decreasing the interaction between the polymer chains. As a result, the adhesive becomes softer, less expensive and easier to produce.

The at least one compound (iii) also enables other functionalities to be incorporated into the adhesive composition, without affecting the initial peel force or switch performance. Molecules containing these functionalities would otherwise be mixed in to the adhesive polyurethane composition as small molecules. Thus, by adding them into the polyurethane network, the cytotoxicity of the adhesive is reduced. For example, the at least one compound (iii) may comprise or consist of one or more photoinitiators that contain a nucleophilic functional group containing an active hydrogen atom, e.g. a hydroxy group, for attachment to the polyisocyanate component (i). For the purposes of the invention, a photoinitiator is considered a molecule that initiates a curing reaction but is not itself curable. Accordingly, a photoinitiator that is bound to the polyisocyanate is taken to be a compound (iii) which does not include a functional group that is curable by free radical polymerisation.

Other examples of compounds which may be incorporated into the adhesive composition of the invention as compound (iii) include biocides and catalysts.

Typically, the at least one compound (iii) comprises a single nucleophilic group containing an active hydrogen atom. However, it is not excluded that the at least one compound (iii) may include more than one nucleophilic functional group containing an active hydrogen atom, in which case the compound (iii) may link together two polyisocyanate molecules.

### Adhesive compositions

The relative amounts of the polymer component (A) and the cross-linking component (B) are defined herein by reference to the molar ratio of unsubstituted isocyanate groups in the cross-linking component (B) to the available nucleophilic groups in the polymer component (A). As noted above, it has been found that the cytotoxicity of the adhesive is reduced when the molar ratio of unsubstituted isocyanate groups in the cross-linking component (B) to the nucleophilic groups in the polymer component (A) is at least 0.8.

Preferably, the molar ratio of unsubstituted isocyanate groups in the cross-linking component (B) to nucleophilic groups containing an active hydrogen atom in the polymer component (A) is at least 0.81, or at least 0.82, or at least 0.83, or at least 0.84, or at least 0.85, or at least 0.86, or at least 0.87, or at least 0.88, or at least 0.89, or at least 0.90, or at least 0.91, or at least 0.92, or at least 0.93, or at least 0.94, or at least 0.95.

If compound (ii) is used in the reaction to obtain the cross-linking component (B), the adhesive polyurethane preferably comprises from 0.05 to 1 meq/g, preferably from 0.06 to 0.5 meq/g, preferably from 0.08 to 0.4 meq/g, preferably from 0.1 to 0.3 meq/g, preferably from 0.12 to 0.25 meq/g, preferably from 0.15 to 0.2 meq/g of the functional group that is curable by free-radical polymerisation. As noted above, while high levels of curable groups are viable, the benefit in switching performance is small and adds to the cost of producing the adhesive without any substantial performance benefit.

The reactants used to form the adhesive compositions of the invention are preferably substantially free of water. Water may for instance be present as a minor impurity in the polyol component (A) or in the compounds (ii) and (iii). Water reacts with the isocyanate groups of the cross-linking component (B) and therefore reduces the number of unsubstituted isocyanate functions in the cross-linking component (B) that are available to react with the nucleophilic functional groups in the polymer component (A). For avoidance of doubt, references herein to the molar ratio of unsubstituted isocyanate functions in the cross-linking component (B) to nucleophilic functional groups containing an active hydrogen atom in the polymer component (A) take into account the degree of substitution of the polyisocyanate component (i) by water, and the content of unsubstituted isocyanate functions is adjusted accordingly.

Preferably, the maximum degree of substitution of the polyisocyanate component (i) by water is no more than 0.1, preferably no more than 0.08, preferably no more than 0.06, preferably no more than 0.05, preferably no more than 0.04. Taking into account that water is a di-functional molecule, the total degree of substitution of the polyisocyanate component (i) by water is calculated as follows: $degree of substitution by water = \frac{2 \times total moles water}{total moles isocyanate groups in polyisocyanate component \left(i\right)}$

### Photoinitiators

For adhesive compositions of the invention which are switchable and contain compound (ii) in the cross-linking component (B), the adhesive polyurethane composition preferably comprises a photoinitiator, preferably from 0.05 to 5 wt% of a photoinitiator, preferably from 0.1 to 5 wt% of a photoinitiator preferably from 0.2 to 2 wt% of a photoinitiator. These particular ranges of photoinitiator enable the curable groups to react with one another upon activation, and allow the adhesive to achieve desirable switch times.

The photoinitiator may be mixed into the adhesive composition and/or bound to the polymeric chains of the adhesive composition. Optionally, the photoinitiator may be used as a compound (iii), provided that it has a suitable nucleophilic functional group for attachment to the polyisocyanate component and so long as the photoinitiating function is not impeded thereby.

The photoinitiator may be any species which is capable of producing radical species under mild conditions, e.g. UV or visible light, in order to promote free-radical initiated polymerization of the curable functional groups of compound (ii).

Preferably, the photoinitiator is reactive to UV radiation having wavelength in the range from 200 to 400 nm), preferably UVA radiation (315 to 400 nm). UVA is particularly preferred for medical applications and other applications requiring exposure of humans or animals to the UV radiation. The range of 200 to 400 nm is referred to herein as "long wavelength UV".

The photoinitiator may alternatively produce radical species upon exposure to visible light, but products that are curable upon exposure to visible light require careful handling and/or require additional visible light occlusive materials to be incorporated in the product to avoid premature switching of the adhesive. The visible light occlusive material needs to be removed from the product at the appropriate time, when switching is desired.

The photoinitiator reactive to UV may be selected from any of the conventional photoinitiators known in the art. For example, the photoinitiator reactive to UV may suitably be selected from the group consisting of benzoin and derivatives (e.g. the ethyl, isopropyl or isobutyl ethers of benzoin); benzophenone and derivatives (e.g. 4-phenylbenzophenone); acetophenone and 4-phenoxyacetophenone; 2-methyl-1-[4-(methylthio)phenyl]-2-(4-morpholinyl)-1-propanone, 2-benzyl-2-(dimethylamino)-1-[4-(4-morpholinyl)phenyl]-1-butanone; 2-dimethylamino-2-(4-methyl-benzyl)-1-(4-morpholin-4-yl-phenyl)-butan-1-one; 2-ethyl anthraquinone; benzil dimethyl ketal; 1-[4-(2-hydroxyethoxyl)-phenyl]-2-hydroxy-2-methylpropanone; 2-hydroxy-1-[4-[4-(2-hydroxy-2-methylpropionyl)benzyl)-phenyl)-2-methylpropan-1-one; 2-hydroxy-2-methyl propiophenone; and ethyl-4-(dimethylamino) benzoate.

Suitable free radical initiators for visible light activation include titanocene photoinitiators; dye/co-initiator systems, e.g., thionine/triethanolamine; dye/peroxide systems and 1,2-diketone/co-initiator systems, e.g., camphorquinone/tertiary amine. Examples of visible light photoinitiators are: phenanthrenequinone; titanocenes; and bis(2,4,6-trimethyl-benzoyl)-phenylphosphineoxide.

### Solvent

The adhesive composition may further comprise a solvent. The solvent must be a non-protic solvent so that it does not react with the isocyanate groups of the polyisocyanate component (i). Preferably, the solvent has low toxicity, preferably the solvent is non-toxic. An example of a preferred solvent is ethyl acetate.

### Stabilizer

If the adhesive composition comprises curable functional groups, it may also contain a stabilizer. As used herein, the term "stabilizer" refers to a substance that is added to the adhesive composition to scavenge free-radicals so as to prevent premature reaction of the curable functional groups in the adhesive during manufacture and/or storage of the adhesive composition. These substances are well-known in the art of curable materials and may also be referred to as antioxidants.

Examples of suitable stabilizers include 1-piperidinyloxy-4,4'-[1,10-dioxo-1,10-decanediyl)bis(oxy)]bis[2,2,6,6-tetramethyl] and phenolic derivatives such as methoxyphenol, di-tert-butyl-4-methylphenol, and pentaerythritol tetrakis(3-(3,5-di-tert-butyl-4-hydroxy-phenyl)propionate).

### Optional Constituents

If the adhesive composition comprises curable functional groups, it may also contain a photo-sensitizer. Since a sensitizing species often absorbs energy in a different part of the spectrum from the photoinitiator, more effective use of the light source may be achievable through the incorporation of sensitizers into the composition. Many photo-sensitizers are complex organic molecules, absorbing in the long wavelength UV and/or visible portion of the spectrum.

The adhesive composition may also incorporate scattering particles to increase the effect of irradiation of the adhesive mixture by scattering the irradiating UV or visible light through the thickness of the adhesive mixture. Preferably, the light scattering particles are an inorganic compound such as silica powder, alumina powder, silica-alumina powder or mica powder with particle sizes of the order of 10 nm or greater, typically up to 1 µm.

For switchable adhesive compositions, the reactivity of the composition can be increased by increasing the concentration (meq/g) of curable groups in the adhesive, by using a compound (ii) with two or more curable groups, and/or by using more reactive functionalities in the adhesive, e.g., partly or completely exchanging methacrylate with acrylate (acrylates are more reactive but also slightly more toxic).

The interactions between molecules of the adhesive and thereby the viscosity of the adhesive before switching can be decreased by using bulky groups (e.g., by adding methyl groups or by branching of the polyol component (A)) and/or introducing asymmetry or higher hydrophobicity in the cross-linking component (B). This may be achieved by using polyisocyanate components (i) which have bulky groups e.g., by exchanging hexamethylene diisocyanate with trimethylhexamethylene diisocyanate, exchanging isocyanurate with iminooxadiazinedione, exchanging butanediol with methylpentanediol, exchanging polyethylene glycol with polypropylene glycol, etc. Alternatively, this may also be achieved by using at least one compound (iii) in the cross-linking component (B), wherein the at least one compound (iii) comprises bulky groups.

The adhesive compositions of the invention exhibit a reduction in peel force of after switching of at least 30%, and preferably from 50 to 99%, preferably from 70 to 99% when measured according to the method described below.

In accordance with the first aspect of the invention, there are further provided adhesive compositions according to the following aspects 1-1 to 1-20.

Aspect 1-1: An adhesive polyurethane composition according to the first aspect of the invention, wherein:
(i) X represents a number having a value of at least 2.2;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 to 5 hydroxy groups;
(iv) the polyisocyanate component (i) has an average of 2 to 4 isocyanate functions per molecule;
(v) compound (ii) is present and the degree of substitution of the polyisocyanate component (i) by the compound (ii) is in the range from 0.05 to 0.5, preferably from 0.05 to 0.45;
(vi) the total degree of substitution of the polyisocyanate component (i) by compound (ii) and optional compound (iii) is from 0.25 to 0.65; and
(vii) the molar ratio of unsubstituted isocyanate functions in the cross-linking component (B) to hydroxy groups in the polyol component (A) is at least 0.82.

Aspect 1-2: An adhesive polyurethane composition according to the first aspect of the invention, wherein:
(i) X represents a number having a value of at least 2.4;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 or 4 hydroxy groups;
(iv) the polyisocyanate component (i) has an average of 2.1 to 3.6 isocyanate functions per molecule;
(v) compound (ii) is present and the degree of substitution of the polyisocyanate component (i) by the compound (ii) is in the range from 0.05 to 0.4, preferably from 0.08 to 0.35;
(vi) the total degree of substitution of the polyisocyanate component (i) by compound (ii) and optional compound (iii) is from 0.3 to 0.6; and
(vii) the molar ratio of unsubstituted isocyanate functions in the cross-linking component (B) to hydroxy groups in the polyol component (A) is at least 0.86.

Aspect 1-3: An adhesive polyurethane composition according to the first aspect of the invention, wherein:
(i) X represents a number having a value of at least 2.6;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.2 to 3.4 isocyanate functions per molecule;
(v) compound (ii) is present and the degree of substitution of the polyisocyanate component (i) by the compound (ii) is in the range from 0.08 to 0.3, preferably from 0.1 to 0.25;
(vi) the total degree of substitution of the polyisocyanate component (i) by compound (ii) and optional compound (iii) is from 0.35 to 0.6; and
(vii) the molar ratio of unsubstituted isocyanate functions in the cross-linking component (B) to hydroxy groups in the polyol component (A) is at least 0.9.

Aspect 1-4: An adhesive polyurethane composition according to the first aspect of the invention, wherein:
(i) X represents a number having a value of at least 2.8;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.5 to 3.4 isocyanate functions per molecule;
(v) compound (ii) is present and the degree of substitution of the polyisocyanate component (i) by the compound (ii) is in the range from 0.1 to 0.2, preferably from 0.12 to 0.18;
(vi) the total degree of substitution of the polyisocyanate component (i) by compound (ii) and optional compound (iii) is from 0.4 to 0.6; and
(vii) the molar ratio of unsubstituted isocyanate functions in the cross-linking component (B) to hydroxy groups in the polyol component (A) is at least 0.92.

Aspect 1-5: An adhesive polyurethane composition according to the first aspect of the invention, wherein:
(i) X represents a number having a value of at least 2.2;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 to 5 hydroxy groups;
(iv) the polyisocyanate component (i) has an average of 2 to 4 isocyanate functions per molecule;
(v) compounds (ii) and (iii) are present and the degree of substitution of the polyisocyanate component (i) by the compound (ii) is in the range from 0.05 to 0.5, preferably from 0.05 to 0.45;
(vi) the total degree of substitution of the polyisocyanate component (i) by compound (ii) and compound (iii) is from 0.25 to 0.65; and
(vii) the molar ratio of unsubstituted isocyanate functions in the cross-linking component (B) to hydroxy groups in the polyol component (A) is at least 0.82.

Aspect 1-6: An adhesive polyurethane composition according to the first aspect of the invention, wherein:
(i) X represents a number having a value of at least 2.4;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 or 4 hydroxy groups;
(iv) the polyisocyanate component (i) has an average of 2.1 to 3.6 isocyanate functions per molecule;
(v) compounds (ii) and (iii) are present and the degree of substitution of the polyisocyanate component (i) by the compound (ii) is in the range from 0.05 to 0.4, preferably from 0.08 to 0.35;
(vi) the total degree of substitution of the polyisocyanate component (i) by compound (ii) and compound (iii) is from 0.3 to 0.6; and
(vii) the molar ratio of unsubstituted isocyanate functions in the cross-linking component (B) to hydroxy groups in the polyol component (A) is at least 0.86.

Aspect 1-7: An adhesive polyurethane composition according to the first aspect of the invention, wherein:
(i) X represents a number having a value of at least 2.6;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.2 to 3.4 isocyanate functions per molecule;
(v) compounds (ii) and (iii) are present and the degree of substitution of the polyisocyanate component (i) by the compound (ii) is in the range from 0.08 to 0.3, preferably from 0.1 to 0.25;
(vi) the total degree of substitution of the polyisocyanate component (i) by compound (ii) and compound (iii) is from 0.35 to 0.6; and
(vii) the molar ratio of unsubstituted isocyanate functions in the cross-linking component (B) to hydroxy groups in the polyol component (A) is at least 0.9.

Aspect 1-8: An adhesive polyurethane composition according to the first aspect of the invention, wherein:
(i) X represents a number having a value of at least 2.8;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.5 to 3.4 isocyanate functions per molecule;
(v) compounds (ii) and (iii) are present and the degree of substitution of the polyisocyanate component (i) by the compound (ii) is in the range from 0.1 to 0.2, preferably from 0.12 to 0.18;
(vi) the total degree of substitution of the polyisocyanate component (i) by compound (ii) and compound (iii) is from 0.4 to 0.6; and
(vii) the molar ratio of unsubstituted isocyanate functions in the cross-linking component (B) to hydroxy groups in the polyol component (A) is at least 0.92.

Aspect 1-9: An adhesive polyurethane composition according to the first aspect of the invention, wherein:
(i) X represents a number having a value of at least 2.2;
(ii) the polymer component (A) is a polyether polyol having a weight average molecular weight in the range from 1,000 to 20,000 Dalton, preferably a polyether polyol comprising repeating units derived from ethylene oxide and/or propylene oxide;
(iii) at least 60 mol% of the hydroxy groups in the polyol are found in polyol molecules comprising from 3 to 5 hydroxy groups;
(iv) the polyisocyanate component (i) has an average of 2 to 4 isocyanate functions per molecule;
(v) compounds (ii) and (iii) are present and the degree of substitution of the polyisocyanate component (i) by the compound (ii) is in the range from 0.05 to 0.5, preferably from 0.05 to 0.45;
(vi) the total degree of substitution of the polyisocyanate component (i) by compound (ii) and compound (iii) is from 0.25 to 0.65;
(vii) the molar ratio of unsubstituted isocyanate functions in the cross-linking component (B) to hydroxy groups in the polyol component (A) is at least 0.82;
(viii) the at least one compound (ii) is selected from hydroxy substituted-(C₂-C₂₀)alkyl methacrylate esters and hydroxy substituted-(C₂-C₂₀)alkyl acrylate esters; and
(ix) the at least one compound (iii) is selected from C₁-C₃₀ aliphatic alcohols.

Aspect 1-10: An adhesive polyurethane composition according to the first aspect of the invention, wherein:
(i) X represents a number having a value of at least 2.4;
(ii) the polymer component (A) is a polyether polyol having a weight average molecular weight in the range from 1,000 to 20,000 Dalton, preferably comprising repeating units derived from ethylene oxide and/or propylene oxide;
(iii) at least 60 mol% of the hydroxy groups in the polyol are found in polyol molecules comprising from 3 or 4 hydroxy groups;
(iv) the polyisocyanate component (i) has an average of 2.1 to 3.6 isocyanate functions per molecule;
(v) compounds (ii) and (iii) are present and the degree of substitution of the polyisocyanate component (i) by the compound (ii) is in the range from 0.05 to 0.4, preferably from 0.08 to 0.35;
(vi) the total degree of substitution of the polyisocyanate component (i) by compound (ii) and compound (iii) is from 0.3 to 0.6;
(vii) the molar ratio of unsubstituted isocyanate functions in the cross-linking component (B) to hydroxy groups in the polyol component (A) is at least 0.86;
(viii) the at least one compound (ii) is selected from hydroxy substituted-(C₂-C₆)alkyl methacrylate esters; and
(ix) the at least one compound (iii) is selected from linear, branched or cyclic C₁-C₁₈ aliphatic alcohols.

Aspect 1-11: An adhesive polyurethane composition according to the first aspect of the invention, wherein:
(i) X represents a number having a value of at least 2.6;
(ii) the polymer component (A) is a polyether polyol having a weight average molecular weight in the range from 1,500 to 10,000 Dalton, preferably comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.2 to 3.4 isocyanate functions per molecule;
(v) compounds (ii) and (iii) are present and the degree of substitution of the polyisocyanate component (i) by the compound (ii) is in the range from 0.08 to 0.3, preferably from 0.1 to 0.25;
(vi) the total degree of substitution of the polyisocyanate component (i) by compound (ii) and compound (iii) is from 0.35 to 0.6;
(vii) the molar ratio of unsubstituted isocyanate functions in the cross-linking component (B) to hydroxy groups in the polyol component (A) is at least 0.9;
(viii) the at least one compound (ii) is selected from 2-hydroxyethyl methacrylate, 2-hydroxypropyl methacrylate, 2-hydroxyethyl acrylate, 2-hydroxypropyl acrylate polypropylene glycol monomethacrylate, and polypropylene glycol monoacrylate; and
(ix) the at least one compound (iii) is selected from linear, branched or cyclic C₁-C₁₂ aliphatic alcohols..

Aspect 1-12: An adhesive polyurethane composition according to the first aspect of the invention, wherein:
(i) X represents a number having a value of at least 2.8;
(ii) the polymer component (A) is a polyether polyol having a weight average molecular weight in the range from 1,000 to 2,500 Dalton, preferably comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.5 to 3.4 isocyanate functions per molecule;
(v) compounds (ii) and (iii) are present and the degree of substitution of the polyisocyanate component (i) by the compound (ii) is in the range from 0.1 to 0.2, preferably from 0.12 to 0.18;
(vi) the total degree of substitution of the polyisocyanate component (i) by compound (ii) and compound (iii) is from 0.4 to 0.6;
(vii) the molar ratio of unsubstituted isocyanate functions in the cross-linking component (B) to hydroxy groups in the polyol component (A) is at least 0.92;
(viii) the at least one compound (ii) is selected from 2-hydroxypropyl methacrylate, 2-hydroxyethyl methacrylate and mixtures thereof; and
(ix) the at least one compound (iii) is selected from branched C₃-C₁₂ aliphatic alcohols.

Aspect 1-13: An adhesive polyurethane composition according to the first aspect of the invention, wherein:
(i) X represents a number having a value of at least 2.2;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 to 5 hydroxy groups;
(iv) the polyisocyanate component (i) has an average of 2 to 4 isocyanate functions per molecule;
(v) compound (ii) is absent;
(vi) the total degree of substitution of the polyisocyanate component (i) by compound (iii) is from 0.25 to 0.65; and
(vii) the molar ratio of unsubstituted isocyanate functions in the cross-linking component (B) to hydroxy groups in the polyol component (A) is at least 0.82.

Aspect 1-14: An adhesive polyurethane composition according to the first aspect of the invention, wherein:
(i) X represents a number having a value of at least 2.4;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 or 4 hydroxy groups;
(iv) the polyisocyanate component (i) has an average of 2.1 to 3.6 isocyanate functions per molecule;
(v) compound (ii) is absent;
(vi) the total degree of substitution of the polyisocyanate component (i) by compound (iii) is from 0.3 to 0.6; and
(vii) the molar ratio of unsubstituted isocyanate functions in the cross-linking component (B) to hydroxy groups in the polyol component (A) is at least 0.86.

Aspect 1-15: An adhesive polyurethane composition according to the first aspect of the invention, wherein:
(i) X represents a number having a value of at least 2.6;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.2 to 3.4 isocyanate functions per molecule;
(v) compound (ii) absent;
(vi) the total degree of substitution of the polyisocyanate component (i) by compound (iii) is from 0.35 to 0.6; and
(vii) the molar ratio of unsubstituted isocyanate functions in the cross-linking component (B) to hydroxy groups in the polyol component (A) is at least 0.9.

Aspect 1-16: An adhesive polyurethane composition according to the first aspect of the invention, wherein:
(i) X represents a number having a value of at least 2.8;
(ii) the polymer component (A) is a polyol, preferably a polyether polyol, preferably a polyether polyol comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.5 to 3.4 isocyanate functions per molecule;
(v) compound (ii) is absent;
(vi) the total degree of substitution of the polyisocyanate component (i) by compound (iii) is from 0.4 to 0.6; and
(vii) the molar ratio of unsubstituted isocyanate functions in the cross-linking component (B) to hydroxy groups in the polyol component (A) is at least 0.92.

Aspect 1-17: An adhesive polyurethane composition according to the first aspect of the invention, wherein:
(i) X represents a number having a value of at least 2.2;
(ii) the polymer component (A) is a polyether polyol having a weight average molecular weight in the range from 1,000 to 20,000 Dalton, preferably a polyether polyol comprising repeating units derived from ethylene oxide and/or propylene oxide;
(iii) at least 60 mol% of the hydroxy groups in the polyol are found in polyol molecules comprising from 3 to 5 hydroxy groups;
(iv) the polyisocyanate component (i) has an average of 2 to 4 isocyanate functions per molecule;
(v) compound (ii) is absent;
(vi) the total degree of substitution of the polyisocyanate component (i) by compound (iii) is from 0.25 to 0.65;
(vii) the molar ratio of unsubstituted isocyanate functions in the cross-linking component (B) to hydroxy groups in the polyol component (A) is at least 0.82; and
(viii) the at least one compound (iii) is selected from C₁-C₃₀ aliphatic alcohols.

Aspect 1-18: An adhesive polyurethane composition according to the first aspect of the invention, wherein:
(i) X represents a number having a value of at least 2.4;
(ii) the polymer component (A) is a polyether polyol having a weight average molecular weight in the range from 1,000 to 20,000 Dalton, preferably comprising repeating units derived from ethylene oxide and/or propylene oxide;
(iii) at least 60 mol% of the hydroxy groups in the polyol are found in polyol molecules comprising from 3 or 4 hydroxy groups;
(iv) the polyisocyanate component (i) has an average of 2.1 to 3.6 isocyanate functions per molecule;
(v) compound (ii) is absent;
(vi) the total degree of substitution of the polyisocyanate component (i) by compound (iii) is from 0.3 to 0.6;
(vii) the molar ratio of unsubstituted isocyanate functions in the cross-linking component (B) to hydroxy groups in the polyol component (A) is at least 0.86; and
(viii) the at least one compound (iii) is selected from linear, branched or cyclic C₁-C₁₈ aliphatic alcohols.

Aspect 1-19: An adhesive polyurethane composition according to the first aspect of the invention, wherein:
(i) X represents a number having a value of at least 2.6;
(ii) the polymer component (A) is a polyether polyol having a weight average molecular weight in the range from 1,500 to 10,000 Dalton, preferably comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.2 to 3.4 isocyanate functions per molecule;
(v) compound (ii) is absent;
(vi) the total degree of substitution of the polyisocyanate component (i) by compound (iii) is from 0.35 to 0.6;
(vii) the molar ratio of unsubstituted isocyanate functions in the cross-linking component (B) to hydroxy groups in the polyol component (A) is at least 0.9; and
(viii) the at least one compound (iii) is selected from linear, branched or cyclic C₁-C₁₂ aliphatic alcohols..

Aspect 1-20: An adhesive polyurethane composition according to the first aspect of the invention, wherein:
(i) X represents a number having a value of at least 2.8;
(ii) the polymer component (A) is a polyether polyol having a weight average molecular weight in the range from 1,000 to 2,500 Dalton, preferably comprising repeating units derived from ethylene oxide and/or propylene oxide;
(iii) at least 60 mol% of the hydroxy groups in the polyol are found in polyol molecules comprising from 3 hydroxy groups;
(iv) the polyisocyanate component (i) has an average of 2.5 to 3.4 isocyanate functions per molecule;
(v) compound (ii) is absent;
(vi) the total degree of substitution of the polyisocyanate component (i) by compound (ii) and compound (iii) is from 0.4 to 0.6;
(vii) the molar ratio of unsubstituted isocyanate functions in the cross-linking component (B) to hydroxy groups in the polyol component (A) is at least 0.92; and
(viii) the at least one compound (iii) is selected from branched C₃-C₁₂ aliphatic alcohols.

It is to be understood that any preferred/optional features disclosed herein in relation to the first aspect of the invention that fall within the scope of the above-described aspects 1-1 to 1-20 are also preferred/optional features of the aspects 1-1 to 1-20. Likewise, any features of the dependent claims that fall within the scope of the above-described aspects 1-1 to 1-20 are also to be interpreted as though those claims also depend from aspects 1-1 to 1-20.

### Method of Preparing an Adhesive Composition

According to a second aspect of the invention, there is provided a method of preparing an adhesive composition comprising:
(a) a first step of reacting:
   (i) a polyisocyanate component having an average of 1.8 to 6 isocyanate functions per molecule; and

   with at least one of:
      (ii) at least one compound comprising a functional group that is curable by free-radical polymerisation and further comprising a nucleophilic functional group containing an active hydrogen atom; and
      (iii) at least one compound comprising a nucleophilic functional group containing an active hydrogen atom and which does not include a functional group that is curable by free-radical polymerisation;
   wherein the total degree of substitution of the polyisocyanate component (i) by the compounds (ii) and/or (iii) is in the range from 0.2 to 0.7; to form a cross-linking component (B);
(b) a second step of combining cross-linking component (B) formed in step (a) with a polymer component (A) having a weight average molecular weight in the range of 1,000 to 100,000 Dalton, and containing an average per molecule of X nucleophilic functional groups containing an active hydrogen atom, wherein X represents a number having a value of at least 2,
wherein the amounts of the cross-linking component (B) and the polyol component (A) are selected such that molar ratio of unsubstituted isocyanate functions in the cross-linking component (B) to nucleophilic functional groups containing an active hydrogen atom in the polymer component (A) is at least 0.8.

The method of the second aspect of the invention may be used to produce the adhesive composition of the first aspect of the invention. Accordingly, any feature described as optional or preferred with reference to the first aspect of the invention shall also be understood to constitute an optional or preferred feature with respect to the identity, amounts and ratios of the corresponding components used in the method of the second aspect of the invention. For example, the method of the second aspect of the invention may be used to produce the adhesive composition of any of aspects 1-1 to 1-18 described above.

Step (a) and/or step (b) of the method of preparing an adhesive polyurethane composition may be carried out in the presence of a catalyst. Suitable catalysts include dibutyltin dilaurate, Zirconium (IV) acetylacetonate, dibutyltin 2-ethylhexanoate, Zn(ll) 2-ethyl-1-hexanoate and tertiary amines.

Step (a) and/or step (b) may be carried out in the presence of a solvent. Suitable solvents are non-protic solvents, such as ethyl acetate, toluene and tetrahydrofuran.

Preferably, a photoinitiator is combined with the product of step (a) or with the polyol component prior to step (b). Most preferably, the photoinitiator is combined with the polyol component prior to step (b).

### Adhesive Medical Devices

According to a third aspect of the invention, there is provided an adhesive medical device comprising a layer of an adhesive composition as defined in any of claims 1 to 28 disposed on a first carrier film and a release liner disposed over the adhesive layer.

Said adhesive medical device may comprise a product selected from the group consisting of an adhesive dressing including an absorbent wound pad, a surgical incision drape, a bacterial barrier for covering a wound, and a skin closure device for closing together the edges of a wound.

When the medical product includes a switchable adhesive, suitably, the first carrier film of the medical device may be translucent to UV and/or visible light. Optionally a removable light occlusive layer is laminated to the first carrier film on the surface opposite the adhesive composition.

Exemplary materials for the carrier film for carrying the switchable adhesive composition layer include polyethylene, polypropylene, polyurethane, ethylene/propylene copolymers, ethylene/ethylacrylate copolymers, ethylene/vinyl acetate copolymers, silicone elastomers, polydimethylsiloxanes, neoprene rubber, polyisobutylene, polyacrylates, chlorinated poly-ethylene, polyvinyl chloride, vinyl chloride-vinyl acetate copolymer, crosslinked polymethacrylate polymers (hydrogel), polyvinylidene chloride, poly (ethylene terephthalate), butyl rubber, epichlorohydrin rubbers, ethylenevinyl alcohol copolymers, ethylene- vinyloxyethanol copolymers; silicone copolymers, for example, polysiloxane- polycarbonate copolymers, polysiloxanepolyethylene oxide copolymers, polysiloxane-polymethacrylate copolymers, polysiloxane-alkylene copolymers (e.g., polysiloxane-ethylene copolymers), polysiloxane-alkylenesilane copolymers (e.g., polysiloxane-ethylenesilane copolymers), and the like; cellulose polymers, for example methyl or ethyl cellulose, hydroxy propyl methyl cellulose, and cellulose esters; polycarbonates; polytetrafluoroethylene; and the like. More preferred are medical grade polyethers or polyester polyurethanes, thermoplastic polyester elastomer, perforated polyethylene, polypropylene and PET films, as well as medical grade woven or non-woven materials.

Adhesive wound dressings usually consist of an absorbent pad for absorbing exudates from the dressed wound, the absorbent pad being surrounded by an adhesive area for securing the wound pad in position over the wound. The adhesive area and the wound pad are supported on a carrier film which is often flesh coloured or which may sometimes have an attractive design on its visible surface. The switchable adhesive compositions according to the invention are ideal candidates for use as the adhesive in the adhesive area around the wound pad of an adhesive wound dressing.

Adhesive wound dressings are often applied by lay users in their own homes and are not necessarily applied by medical practitioners. Since few homes have access to suitable UV irradiation equipment, adhesive wound dressings for home use preferably include an adhesive that is curable by visible light. In some cases it is preferable that switchable adhesive wound dressings include an adhesive that is curable by UV light so that the timing of removal of the dressing is controlled by a medical practitioner. In the case that the adhesive is curable by UV light, a removable light occlusive layer may not be necessary.

Adhesive wound dressings intended for domestic use will preferably include light occlusive layers to prevent premature switching of the switchable adhesive composition. The first light occlusive layer is positioned on the opposite side of the carrier film to the adhesive and remains in place until such time as the user wants to remove the adhesive wound dressing. At this point, the first light occlusive layer is removed, exposing the underlying switchable adhesive composition to visible light, resulting in switching of the adhesive composition from a tacky state to a non-tacky or low-tack state. Optionally, a second light occlusive layer may form part of the release liner that is placed on the adhesive side of the dressing. This second light occlusive layer is removed just before the adhesive wound dressing is applied over a wound. The second light occlusive layer may be unnecessary in the case that the adhesive wound dressing is provided in a light occlusive package.

In the case that the adhesive wound dressing includes an adhesive that is curable by UV light, light occlusive layers may not be necessary. Instead, the carrier film of the adhesive wound dressing only needs to be translucent to UV. When it is desired to remove the adhesive wound dressing, a trained medical practitioner shines a suitable UV light source over the adhesive wound dressing to initiate the curing reaction. Within seconds the adhesive loses its tackiness and the wound dressing is easily removed.

The release liner may be selected from any covering material having low surface energy to enable it to be removed easily from the adhesive layer. Suitable release liners include paper and plastic films provided with silicone coatings on the surface that contacts the adhesive composition.

In the case that the adhesive wound dressing does not include an adhesive composition with curable moieties, i.e. wherein component (ii) is not used in the reaction to obtain the cross-linking component (B), the use of a light occlusive layer is not necessary.

According to a fourth aspect of the invention, there is provided a method of treating a wound using an adhesive medical product as defined herein, wherein the method comprises removing the release liner and applying the adhesive medical device to the wound.

The invention will be further described by way of example only and without limitation by reference to the drawings in which:
Figure 1 is a cross-sectional view through an adhesive dressing in accordance with a first embodiment of the invention;
Figure 2 is a perspective view showing the attempted removal from a patient's forearm of an adhesive dressing in accordance with the first embodiment of the invention and includes an enlargement bubble showing in partial cross- section how removal of the adhesive dressing causes the adhesive composition to extrude;
Figure 3 is a perspective view showing the adhesive dressing in accordance with the first embodiment of the invention undergoing irradiation to effect switching of the adhesive;
Figure 4 is a perspective view showing how the adhesive dressing in accordance with the first embodiment of the invention may be easily removed after switching of the adhesive;
Figures 5 and 6 are graphs showing the results of examples 5 to 14.

### DETAILED DESCRIPTION OF THE DRAWINGS

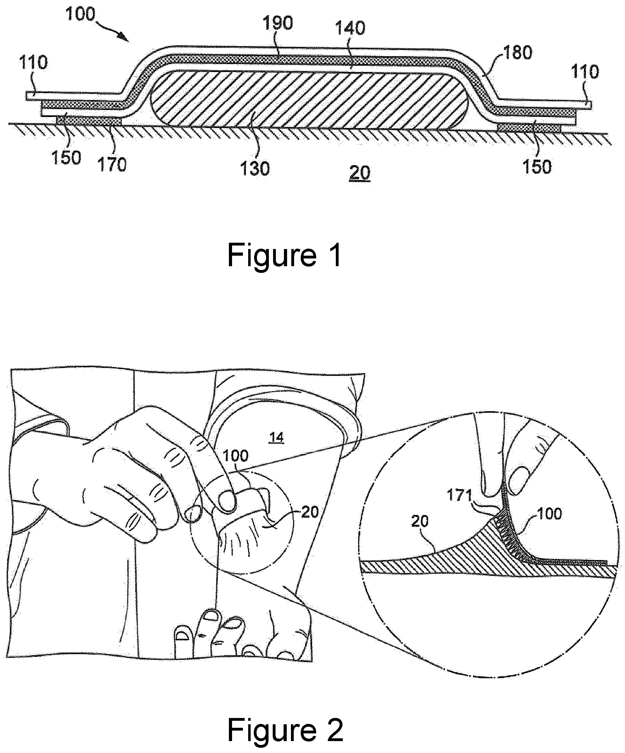

An adhesive medical device using the adhesive polyurethane composition of the present invention will now be described with reference to Figures 1 to 4. The adhesive medical product in this example is an adhesive medical dressing.

Figure 1 is a cross-sectional view through an adhesive medical dressing 100 attached to a patient's skin 20. The adhesive medical dressing 100 is a multi-layer product having the following structure. The dressing 100 comprises a wound facing absorbent layer 130 disposed beneath a protective backing layer 140. At opposed edges 150, the backing layer 140 is provided with an adhesive polyurethane composition 170.

The backing layer 140 is optionally provided with a light occlusive cover layer 180 which is releasably secured to the backing layer 140 by a weak adhesive 190. For ease of removal, the light occlusive cover layer 180 overlaps the backing layer 140 at its edges 110. In the case that the adhesive is a switchable adhesive composition 170 and contains a photoinitiator that is actuated by UV radiation, or in the case where the adhesive composition is not switchable, the light occlusive cover layer 180 may be omitted.

Figure 2 is a perspective view showing the attempted removal of a switchable adhesive dressing 100 from the forearm 14 of a patient, prior to switching of the switchable adhesive composition comprising curable moieties. Before switching, the adhesive composition 171 is very tacky and sticks the adhesive dressing 100 to the patient's skin 20 quite firmly. Hence, when the patient attempts to peel the dressing 100 from the forearm 14, the dressing 100 remains attached to the skin 20 unless the dressing is peeled with some force.

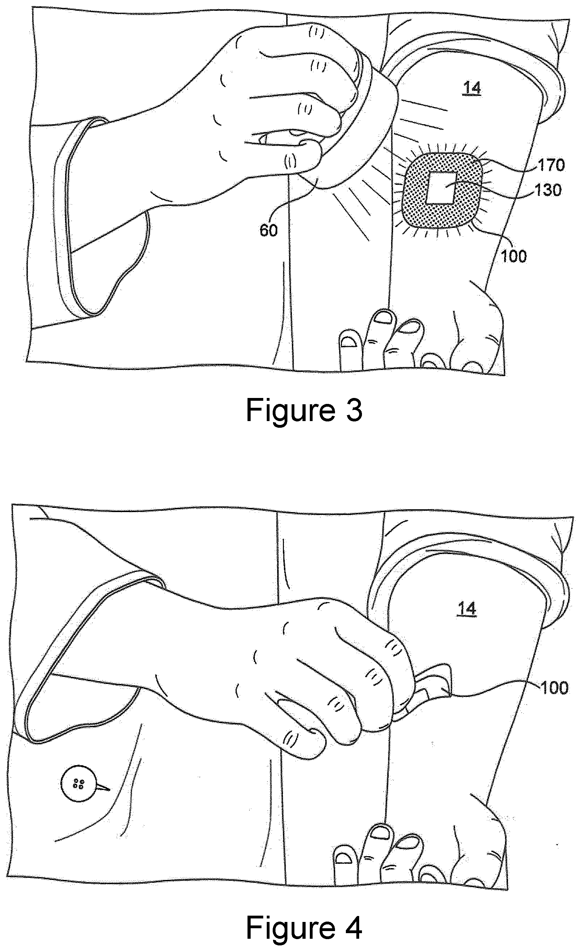

Figure 3 is a perspective view showing the switchable adhesive dressing undergoing irradiation, in this example from a lamp 60, to effect cure of the curable molecules in the adhesive composition 170. The light from the lamp 60 (UV light or visible light, preferably long wavelength UV) causes the photoinitiator in the adhesive composition 170 to generate free radicals that initiate curing of the curable molecules in the adhesive composition. Curing transforms (switches) the adhesive composition 170 from its tacky state to a non-tacky or low-tack state.

Figure 4 is a perspective view showing how, after switching of the adhesive composition, the patient is easily able to remove adhesive dressing 100 from the forearm 14 without the need for excessive force.

### EXAMPLES

### Examples 1-2 - cross-linking component (B)

The reaction was carried out at room temperature under stirring. The solvent B was dried with a 3Å molecular sieve. The components shown below in Table 1 except for the catalyst were then added into a reagent bottle and mixed into a homogeneous solution, after which the catalyst was added. The mixture was left overnight for the reaction to be completed. After confirming by GPC measurements that no unreacted hydroxypropyl methacrylate or 2-ethyl-1-hexanol remained, the isocyanate functional acrylate oligomer was ready to be used.

GPC was carried out by diluting samples with tetrahydrofuran in a ratio of 1:100 and injected in an amount of 20 µl into the injection valve of a Waters HPLC 1515 pump using a flow rate of 1 ml/min of tetrahydrofuran. The instrument was equipped with a Styragel HR1 column connected to a Waters 2414 refractive index detector.

The components shown in Table 1 are as follows:

| | |
|---|---|
| A | Polyisocyanate component (i) |
| B | Solvent |
| C | Catalyst |
| D | Stabilizer |
| E | Compound (ii) |
| F | Compound (iii) |
| G | Water (iv) |

**Table 1**

| **Reagent** | | **Example 1** | **Example 2** |
|---|---|---|---|
| **A** | Baymedix AP536 (3.2 NCO groups per molecule) | 57.62 g | 57.60 g |
| **B** | Ethyl acetate | 9.03 g | 9.00 g |
| **C** | Zirconium (IV) pentanedionate | 0.005 g | 0.004 g |
| **D** | Irganox 1010 | 0.24 g | 0.24 g |
| **E** | Hydroxypropyl methacrylate (ii) | 8.54 g | 8.50 g |
| **F** | 2-Ethyl-1-hexanol (iii) | 16.02 g | 15.60 g |
| **G** | Water (iv) | 0.012 g | 0.012 g |
| Degree of subst. of polyisocyanate component (i) by (ii) and (iii) | | 0.58 | 0.57 |

### Example 3 - Preparation of polyol component (A)

Under protection from ultraviolet sources all components in Table 2 were loaded into a sealable glass jar and mixed until all solid materials had been dissolved using a magnetic stirrer.

The components shown in Tables are as follows:

| | |
|---|---|
| H | Polyol |
| I | Photoinitiator I |
| C | Catalyst |
| D | Stabilizer for preventing premature switch during storage |
| J | Surfactant |
| K | Photoinitiator II |

**Table 2**

| | **Reagent** | **Example 3** |
|---|---|---|
| **H** | Lupranol 2095 (X=3) | 480.00 g |
| **I** | Omnirad 369 | 1.12 g |
| **C** | Borchi Kat 22 | 0.52 g |
| **D** | Songnox 1010 | 0.54 g |
| **J** | BYK378 | 0.16 g |
| **K** | Omnirad 2959 | 12.50 g |

### Example 4 - Preparation of polyol component (A)

The polyol component (A) was prepared in an analogous manner to Example 3, with the following reagents in Table 3.

**Table 3**

| | **Reagent** | **Example 4** |
|---|---|---|
| **H** | Voranol CP6001 (X=3) | 480.00 g |
| **I** | Omnirad 369 | 1.12 g |
| **C** | Borchi Kat 22 | 0.50 g |
| **D** | Songnox 1010 | 0.50 g |
| **J** | BYK378 | 0.16 g |
| **K** | Omnirad 2959 | 12.49 g |

### Examples 5-9 - adhesive polyurethane compositions

Examples 5 to 9 are examples of adhesive compositions in accordance with the present invention formulated to include the cross-linking component (B) from Example 1 and the polymer component (A) from Example 3 in the amounts shown in Table 4. As these compositions comprise curable compound (ii) in the cross-linking component (B), the adhesive compositions are switchable.

Both A and B components in Examples 5 to 9 were loaded into a sealable glass jar and mixed to a homogenous solution using a magnetic stirrer over a period of approximately 10 minutes under protection from ultraviolet sources. The resulting adhesive solution was then spread onto a flexible medical polyurethane film having a removable carrier film (medical film 48938) using a spreader having a gauge of 150 µm. The adhesive coating was then cured in a ventilated fan assisted oven at 130°C for 10 minutes. After this step, the thickness of the adhesive coating was about 70-90 µm.

**Table 4**

| | **Ex. 5** | **Ex. 6** | **Ex. 7** | **Ex. 8** | **Ex. 9** |
|---|---|---|---|---|---|
| **Cross-linking component (B) Ex.1** | 13.02 g | 12.52 g | 12.03 g | 11.84 g | 11.44 g |
| **Polymer component (A) Ex. 3**** | 25.02 g | 25.03 g | 25.02 g | 25.04 g | 25.01 g |
| **NCO/OH*** | 0.969 | 0.932 | 0.895 | 0.881 | 0.852 |

| | | | | | |
|---|---|---|---|---|---|
| * NCO/OH represents the molar ratio of unsubstituted isocyanate functions in the cross-linking component (B) to nucleophilic hydroxyl groups in the polyol component (A) ** the water content of Lupranol 2095 was 0.038 wt% | | | | | |

### Examples 10 to 14 - adhesive polyurethane compositions

Examples 10 to 14 are examples of switchable adhesive compositions in accordance with the present invention formulated to include the cross-linking component (B) from Example 2 and the polymer component (A) from Example 4 in the amounts shown in Table 5.

Adhesives were prepared according to the procedure set out in example 5 to 9.

**Table 5**

| | **Ex. 10** | **Ex. 11** | **Ex. 12** | **Ex. 13** | **Ex. 14** |
|---|---|---|---|---|---|
| **Cross-linking component (B) Ex.2** | 10.39 g | 10.12 g | 9.70 g | 9.52 g | 9.31 g |
| **Polymer component (A) Ex. 4** | 25.02 g | 25.02 g | 25.02 g | 25.10 g | 25.04 g |
| **NCO/OH** | 0.981 | 0.955 | 0.916 | 0.896 | 0.878 |

| | | | | | |
|---|---|---|---|---|---|
| * NCO/OH represents the molar ratio of unsubstituted isocyanate functions in the cross-linking component (B) to nucleophilic hydroxyl groups in the polyol component (A) ** the water content of Lupranol 2095 was 0.038 wt% | | | | | |

### Peel force measurements

Peel force before and after switching was determined for each of the adhesives of Examples 5 to 14. In preparation for peel force measurements, a very easy release liner was transferred to the exposed side of the adhesive. The removable carrier film was then removed from the medical film and replaced by a high adhesion PET tape. The PET tape is fixed to the medical film in order to cancel out the elasticity effect of the medical film on the measured peel force.

Peel force was determined after a dwell time of 20 minutes using an Instron 5943 testing rig, equipped with a 100 N load cell, according to FINAT test method FTM1, with the exception that stainless steel was used as the substrate surface and that a peeling rate of 100 mm/min, crosshead speed 200 mm/s, was used in order to collect all of the necessary data within the time frame of one peel force measurement.

Adhesive switching was achieved by exposing the adhesive (adhered to the steel plate) to light through the PET tape and medical film backing with a light intensity of approximately 50W/m2 from a XeLED-Ni3UV-R4-365-E27-SS lamp having a narrow spectrum around 365 nm. Switching times for the different coatings were measured as the time between the starting time of irradiation and the time when the substantially instantaneous loss of tack occurred, during a continuous peel strength test of about 1.5 minutes (i.e., the adhesive was peeled for a period of time whilst being irradiated). The peel force measurement was continued under irradiation until the peel force reached a plateau value, which typically occurred 5-10 seconds after the switch time. Peel force at the plateau value and switch times were measured in quadruple and the mean average values of switch time and peel force (before and after switch) are reported in Table 6.

### Cytotoxicity evaluation

The cytotoxicity of the adhesive forming Examples is measured according to the guidelines of ISO 10993-5, Biological evaluation of Medical Devices, Part 5: Test for In Vitro Cytotoxicity, standard for the elution test is displayed in Table 6 below. To determine if a sample has passed the test or not the measured viability must coincide or exceed the value of 70%.

Results of the peel force measurements are shown in Table 6 alongside the viability results from the cytotoxicity measurements. Clean adhesive failure between the substrate steel surface and the adhesive is achieved if not otherwise mentioned.

**Table 6**

| **Example** | **Peel force before switch (N/25mm)** | **Peel force after switch (N/25mm)** | **Switch time (s)** | **Theoretical calculated ratio* (NCO index)** | **Viability (%)** |
|---|---|---|---|---|---|
| **5** | 3.84 | 0.074 | 4.8 | 0.969 | 87.00 |
| **6** | 4.39 | 0.082 | 4.4 | 0.932 | 81.25 |
| **7** | 4.88 | 0.081 | 4.2 | 0.895 | 78.25 |
| **8** | 4.76 | 0.070 | 4.0 | 0.881 | 77.10 |
| **9** | 5.00 | 0.069 | 3.8 | 0.852 | 76.35 |
| **10** | 4.17 | 0.079 | 4.5 | 0.981 | 75.85 |
| **11** | 5.72 | 0.090 | 4.1 | 0.955 | 72.95 |
| **12** | 6.38 | 0.072 | 3.9 | 0.916 | 69.60 |
| **13** | 6.08 | 0.078 | 3.5 | 0.896 | 68.40 |
| **14** | 8.87 | 0.068 | 4.1 | 0.878 | 67.65 |

From Table 6, it can be seen that a reduction in peel force of around 99% is obtained in all cases and that the switched peel force seems to be independent on the unswitched one.

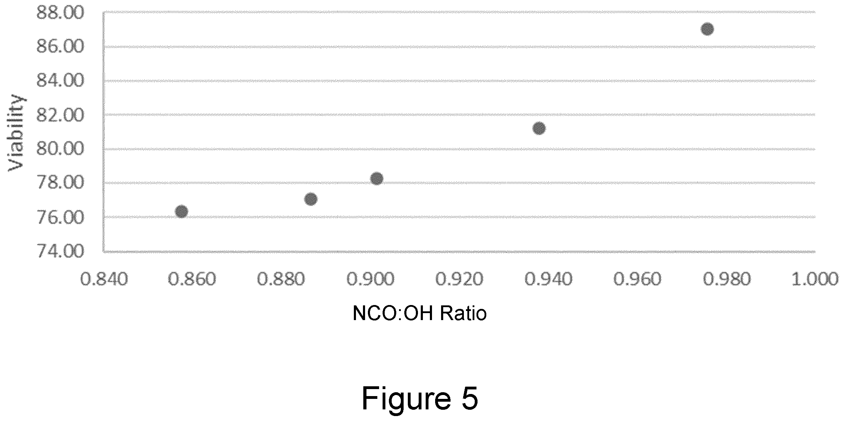

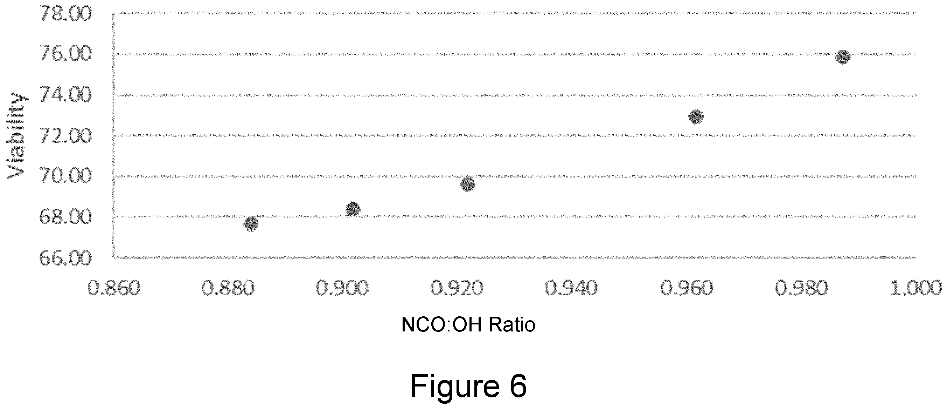

The results of the viability measurements versus their NCO index for adhesives in Example 5-9 and 10-14 are shown graphically in Figures 5 and 6, respectively.

Figure 5 demonstrates the cytotoxicity test results for adhesives based on the triol Lupranol 2095, having different A to B component ratios.

Figure 6 demonstrates the cytotoxicity test results for adhesives based on the triol Voranol CP6001, having different A to B component ratios.

As can be seen in Figures 5 and 6 there is a strong correlation between the viability and the NCO index and since the changes in the concentration of the included components are relatively small at the present ratios, without wishing to be bound by theory, it is believed that the increase in viability of the NCO index is due to the decrease in the remaining unreacted polyol hydroxy groups. A plausible reason for the toxicity of unbound polyol would be that the polyol has some surface activity and thereby disrupts cell membranes, similar to many other detergents, like for instance ordinary soap.

### Example 15 - cross-linking component (B)

A cross-linking component (B) was prepared in an analogous manner to Examples 1 and 2, using the following components.

**Table 7**

| | **Reagent** | **Example 15** |
|---|---|---|
| **A** | Baymedix AP501 (i) (Y=2) | 51.94 g |
| **8** | Ethyl acetate | 11.00 g |
| **C** | Zirconium (IV) pentanedionate | 0.007 g |
| **D** | Irganox 1010 | 0.24 g |
| **E** | Hydroxypropyl methacrylate (ii) | 5.02 g |
| **F** | 2-Ethyl-1-hexanol (iii) | 0 g |
| Degree of substitution of polyisocyanate component (i) by (ii) and (iii) | | 0.22 |

### Examples 16 and 17 - adhesive polyurethane compositions

The adhesive polyurethane compositions of Examples 16 and 17 were prepared in an analogous manner to Examples 5 to 9.

**Table 8**

| | **Ex. 16** | **Ex. 17** |
|---|---|---|
| **Cross-linking component (B) Ex.15** | 8.74 g | 9.08 g |
| **Polymer component (A) Ex. 3** | 20.05 g | 20.05 g |

Peel force and viability measurements were obtained, and the values are shown on Table 9.

**Table 9**

| **Example** | **Peel force before switch (N/25mm)** | **Peel force after switch (N/25mm)** | **Switch time (s)** | **Theoretical calculated ratio* (NCO index)** | **Viability (%)** |
|---|---|---|---|---|---|
| **16** | 5.08 | 0.067 | 4.3 | 0.972 | 77.50 |
| **17** | 5.01 | 0.074 | 4.3 | 1.011 | 80.00 |

### Examples 18-20 - cross-linking component (B)

The reaction was carried out at room temperature under stirring. The solvent B was dried with a 3Å molecular sieve. The components shown below in Table 1 except for the catalyst were then added into a reagent bottle and mixed into a homogeneous solution, after which the catalyst was added. The mixture was left overnight for the reaction to be completed. After confirming by GPC measurements that no unreacted hydroxypropyl methacrylate or 2-ethyl-1-hexanol remained, the isocyanate functional acrylate oligomer was ready to be used.

GPC was carried out by diluting samples with tetrahydrofuran in a ratio of 1:100 and injected in an amount of 20 µl into the injection valve of a Waters HPLC 1515 pump using a flow rate of 1 ml/min of tetrahydrofuran. The instrument was equipped with a Styragel HR1 column connected to a Waters 2414 refractive index detector.

After confirming that all the hydroxy-containing substances have reacted the NCO Content was measured according to the ASTM standard D 2572-97 (Reapproved 2003).

The components shown in Table 10 are as follows:

| | |
|---|---|
| A | Polyisocyanate component (i) |
| B | Solvent |
| C | Catalyst |
| D | Stabiliser |
| E | Compound (ii) |
| F | Compound (iii) |
| G | Water (iv) |

**Table 10**

| **Reagent** | | **Ex. 18** | **Ex. 19** | **Ex. 20** |
|---|---|---|---|---|
| **A** | Baymedix AP536 (3.2 NCO groups per molecule) | 58.06g | 60.23g | 60.34g |
| **B** | Ethyl acetate | 10.00g | 10.00g | 10.00g |
| **C** | Zirconium (IV) pentanedionate | 0.014g | 0.009g | 0.010g |
| **D** | Irganox 1010 | 0 | 0 | 0 |
| **F** | Butyl lactate (iii) | 15.16g | 0 | 0 |
| **F** | 2-Phenylethanol (iii) | 0 | 13.05g | 23.95g |
| **F** | 2-Ethyl-1-hexanol (iii) | 10.02g | 10.55g | 0 |
| **G** | Water (iv) | 0.013g | 0.012g | 0.012g |
| Degree of subst. of polyisocyanate component (i) by (iii) | | 0.57 | 0.57 | 0.60 |

### Example 21 - Preparation of polyol component (A)

Under protection from ultraviolet sources, all components in Table 11 were loaded into a sealable glass jar and mixed using a magnetic stirrer until all solid materials had been dissolved.

The components shown in Tables 11 are as follows:

| | |
|---|---|
| H | Polyol |
| I | Photoinitiator I |
| B | Solvent |
| C | Catalyst |
| D | Stabilizer for preventing premature switch during storage |
| J | Surfactant |
| K | Photoinitiator II |

**Table 11**

| | **Reagent** | **Ex. 21** |
|---|---|---|
| **H** | Voranol CP6001 (X=3) | 452.5 g |
| **I** | Omnirad 369 | 0 g |
| **B** | Ethyl acetate | 0 g |
| **C** | Borchi Kat 22 | 0.34 g |
| **D** | Songnox 1010 | 0 g |
| **J** | BYK378 | 0 g |
| **K** | Omnirad 2959 | 0 g |

### Examples 22-24 - adhesive polyurethane compositions

Examples 22-24 are adhesive compositions in accordance with the present invention formulated to include the cross-linking component (B) from Example 18 and the polymer component (A) from Example 21 in the amounts shown in Table 12. As these compositions comprise curable compound (ii) in the cross-linking component (B), the adhesive compositions are switchable.

Both A and B components in Examples 22 to 24 were loaded into a sealable glass jar and mixed to a homogenous solution using a magnetic stirrer over a period of approximately 10 minutes under protection from ultraviolet sources. The resulting adhesive solution was then spread onto a flexible medical polyurethane film having a removable carrier film (medical film 48938) using a spreader having a gauge of 150 µm. The adhesive coating was then cured in a ventilated fan assisted oven at 130°C for 10 minutes. After this step, the thickness of the adhesive coating was about 70-90 µm.

**Table 12**

| | **Ex. 22** | **Ex. 23** | **Ex. 24** |
|---|---|---|---|
| **Cross-linking component (B) Ex.18** | 5.57 g | 5.28 g | 5,15 g |
| **Polymer component (A) Ex. 21** | 15.08 g | 15.19 g | 15,03 g |
| **NCO/OH** | 1.048 | 0.987 | 0.973 |

### Examples 25-27 - adhesive polyurethane compositions

Examples 25 to 27 are examples of switchable adhesive compositions in accordance with the present invention formulated to include the cross-linking component (B) from Example 19 and the polymer component (A) from Example 21 in the amounts shown in Table 13.

Adhesives were prepared according to the procedure set out in example 22 to 24.

**Table 13**

| | **Ex. 25** | **Ex. 26** | **Ex. 27** |
|---|---|---|---|
| **Cross-linking component (B) Ex.19** | 4.76 g | 4.65 g | 4.54 g |
| **Polymer component (A) Ex. 21** | 15.16 g | 15.17g | 15.15 g |
| **NCO/OH** | 0.919 | 0.898 | 0.878 |

### Examples 28-29 - adhesive polyurethane compositions

Examples 28 to 29 are examples of switchable adhesive compositions in accordance with the present invention formulated to include the cross-linking component (B) from Example 20 and the polymer component (A) from Example 21 in the amounts shown in Table 14.

Adhesives were prepared according to the procedure set out in example 22 to 24.

**Table 14**

| | **Ex. 28** | **Ex. 29** |
|---|---|---|
| **Cross-linking component (B) Ex.20** | 6.13 g | 5.97 g |
| **Polymer component (A) Ex. 21** | 15.12 g | 15.19g |
| **NCO/OH** | 0.986 | 0.948 |

### Peel force measurements

Peel force before and after switching was determined for each of the adhesives of Examples 22 to 29. In preparation for peel force measurements, a release liner was transferred to the exposed side of the adhesive. The removable carrier film was then removed from the medical film and replaced by a high adhesion PET tape. The PET tape is fixed to the medical film in order to cancel out the elasticity effect of the medical film on the measured peel force.

Peel strengths were determined after a dwell time of 20 minutes using an Instron 5943 testing rig, equipped with a 100 N load cell, according to FINAT test method FTM1, with the exception that stainless steel was used as the substrate surface and that a peeling rate of 100 mm/min, crosshead speed 200 mm/s, was used in order to collect all of the necessary data within the time frame of one peel force measurement. The results are shown in Table 15.

**Table 15**

| **Example** | **Peel force before switch (N/25mm)** | **Actual* calculated ratio (NCO index)** | **Theoretical** calculated ratio (NCO index)** |
|---|---|---|---|
| **22** | 2.65 | 1.006 | 1.048 |
| **23** | 3.44 | 0.947 | 0.987 |
| **24** | 4.15 | 0.934 | 0.973 |
| **25** | 2.45 | 0.881 | 0.919 |
| **26** | 3.35 | 0.860 | 0.898 |
| **27** | 3.74 | 0.842 | 0.878 |
| **28** | 3.46 | 0.958 | 0.986 |
| **29** | 3.71 | 0.921 | 0.948 |

From Table 5, it can be seen that a decrease in the NCO index, as shown before, leads to an increased peel force for all three series which means that the peel force can be regulated by the NCO index. It should also be pointed out that the highest peel force in each series, Example 24, 27 and 29, represent the maximum achievable peel force value for each of the combined A and B-components that can be reached without cohesive failure. Furthermore, the real NCO index of the final adhesive can be expected to be somewhat lower than the actual calculated one since the isocyanate functions also in this case can be expected to react internally as well as with ambient humidity.

### Materials

The following materials were used in the examples described above.

| **Material** | **Description** | **Supplier** |
|---|---|---|
| Voranol CP6001 [Polyol component A] | Tri-functional hydroxy-terminated polyether polyol; equivalent weight per hydroxyl group of ca. 2000 Da. | Dow Europe GMBH |
| | X = 3 | |
| Lupranol 2095 [Polyol component A] | Tri-functional hydroxy-terminated polyether polyol; equivalent weight per hydroxyl group of ca. 1600 Da. | BASF Polyurethanes GmbH |
| | X = 3 | |
| Baymedix AP501 [Polyisocyanate component (i)] | Aliphatic NCO terminated prepolymer based on hexamethylene diisocyanate NCO content 12.8 w/w% | Covestro AG |
| | NCO functions per molecule = 2 | |
| Baymedix AP536 [Polyisocyanate component (i)] | Trimerized hexamethylene diisocyanate NCO content 23 w/w% | Covestro AG |
| | NCO functions per molecule = 3.2 | |
| Hydroxypropyl methacrylate [Compound (ii)] | Hydroxy containing methacrylate | GEO Specialty Chemicals |
| Borchi Kat 22 | Polyurethane catalyst | Borchers |
| Phenyl ethanol [Compound (iii)] | Hydroxy containing polar component | Merck KGaA |
| Butyl lactate [Compound (iii)] | Hydroxy containing polar component | Merck KGaA |
| Zirconium (IV) pentanedionate | Polyurethane catalyst | Merck KGaA |
| BYK 378 | Surfactant | BYK-Chemie GMBH |
| Songnox 1010 | Pentaerythritol Tetrakis(3-(3,5-di-tert-butyl-4-hydroxyphenyl) propionate (antioxidant) | IGM Resins |
| Omnirad 2959 | 1-[4-(2-Hydroxyethoxyl)-phenyl]-2-hydroxy-2-methylpropanone | IGM Resins |
| Omnirad 369 | 2-Benzyl-2-(dimethylamino)-4'-morpholinobutyrophenone (photoinitiator) | IGM Resins |
| Code 48938 | Medical film | Shanghai ISO Medical Products Co. Ltd. |
| Stainless steel panels | Panels for peel test measurements | Chemlnstrument Inc. |
| Release liner | Transparent PET Release film, 50 µm, silicone 1720, quality 1876 | Huhtamaki Oyj |
| 2-Ethyl-1-hexanol [compound (iii)] | Fatty alcohol | BUFA Chemikalien GmbH & Co. KG |
| Ethyl acetate | Solvent | Solveco AB |
| Molecule sieve 3Å | Water scavenger | Merck KGaA |

## Claims

1. An adhesive composition comprising the reaction product of:
(A) a polymer component having a weight average molecular weight in the range of 1,000 to 100,000 Dalton, and containing an average per molecule of X nucleophilic functional groups containing an active hydrogen atom, wherein X represents a number having a value of at least 2; and
(B) a cross-linking component obtained by reacting:
(i) a polyisocyanate component having an average of 1.8 to 6 isocyanate functions per molecule;
with at least one of:
(ii) at least one compound comprising a functional group that is curable by free-radical polymerisation and further comprising a nucleophilic functional group containing an active hydrogen atom; and
(iii) at least one compound comprising a nucleophilic functional group containing an active hydrogen atom and that does not include a functional group that is curable by free-radical polymerisation;
wherein the total degree of substitution of the polyisocyanate component (i) by the compounds (ii) and (iii) is in the range from 0.1 to 0.7; and wherein the molar ratio of unsubstituted isocyanate functions in the cross-linking component (B) to nucleophilic functional groups containing an active hydrogen atom in the polymer component (A) is at least 0.8.

2. An adhesive composition according to claim 1, wherein X has a value of at least 2.2, or at least 2.4, or at least 2.6, or at least 2.8, or at least 3, or at least 3.2.

3. An adhesive composition according to any claim 1 or claim 2, wherein the polymer component is a polyol, preferably a polyol comprising an average of 2.8 to 5 hydroxy groups per molecule.

4. An adhesive composition according to claim 3, wherein at least 50 mol% of the hydroxy groups in the polyol are found in polyol molecules comprising from 3 to 5 hydroxy groups, or wherein at least 60 mol% of the hydroxy groups in the polyol are found in polyol molecules comprising from 3 to 5 hydroxy groups, or wherein at least 70 mol% of the hydroxy groups in the polyol are found in polyol molecules comprising from 3 to 5 hydroxy groups, or wherein at least 80 mol% of the hydroxy groups in the polyol are found in polyol molecules comprising from 3 to 5 hydroxy groups, or wherein at least 90 mol% of the hydroxy groups in the polyol are found in polyol molecules comprising from 3 to 5 hydroxy groups.

5. An adhesive polyurethane composition according to any preceding claim, wherein the polymer component (A) is a polyol selected from hydroxy-terminated polyethers and hydroxy-terminated polyesters;
preferably wherein the hydroxy-terminated polyether is an alkoxylated derivative of compound comprising from 3 to 5 hydroxy groups, or a mixtures thereof;
preferably wherein the compound comprising from 3 to 5 hydroxy groups is selected from glycerol, trimethylolpropane, erythritol, pentaerythritol, pentane-1,2,4,5-tetrol, dextrose and mixtures thereof, preferably wherein the alkoxylated derivatives are ethoxylated derivates, propoxylated derivatives, or ethoxylated-co-propoxylated derivatives.

6. An adhesive polyurethane composition according to any preceding claim, wherein the polymer component has a weight average molecular weight in the range of 1,000 to 50,000 Dalton, preferably in the range of 1,000 to 20,000 Dalton, preferably in the range of 1,500 to 10,000 Dalton;
preferably wherein the polymer component has an equivalent weight per nucleophilic functional group of from 200 to 5,000, preferably from 500 to 2,500, preferably from 1,000 to 2,000.

7. An adhesive polyurethane composition according to any preceding claim, wherein the polyisocyanate component (i) comprises an average of 2 to 4 isocyanate functions per molecule, preferably 2.1 to 3.6 isocyanate functions per molecule, preferably 2.1 to 3.5 isocyanate functions per molecule, preferably 2.2 to 3.4 isocyanate functions per molecule, preferably 2.5 to 3.4 isocyanate functions per molecule;
preferably wherein the polyisocyanate component (i) comprises a trimerized diisocyanate of the formula D(R-NCO)₃, wherein D represents a ring structure selected from isocyanurate and iminooxadiazindione or a branched structure selected from biuret and allophanate and mixtures thereof, and each R independently represents a straight chain, branched or cyclic alkylene group having from 2 to 15 carbon atoms or an aryl group having from 6 to 20 carbon atoms, preferably wherein the trimerized diisocyanate is trimerized hexamethylene diisocyanate.

8. An adhesive composition according to any preceding claim, wherein the cross-linking component (B) is obtained by reacting the polyisocyanate component (i) with compound (ii) and optionally with the compound (iii);
preferably wherein the degree of substitution of the polyisocyanate component (i) by the compound (ii) is in the range from 0.05 to 0.5; or from 0.05 to 0.45, or from 0.05 to 0.4, or from 0.8 to 0.35, or from 0.8 to 0.3, or from 0.1 to 0.25, or from 0.1 to 0.2, or from 0.12 to 0.18;
preferably wherein the total degree of substitution of the polyisocyanate component (i) by the compounds (ii) and (iii) is in the range from 0.15 to 0.65, or in the range from 0.2 to 0.6 or in the range from 0.25 to 0.6, or in the range from 0.3 to 0.6.

9. An adhesive polyurethane composition according to claim 8, wherein the at least one compound (ii) comprises an olefin moiety as the functional group that is curable by free-radical polymerisation; preferably wherein the at least one compound (ii) is selected from hydroxy-substituted acrylate esters, hydroxy-substituted methacrylate esters, and mixtures thereof; preferably wherein the at least one compound (ii) is selected from hydroxy-(C₂-C₂₀)alkyl-substituted methacrylate esters, polyalkoxylated monomethacrylate esters containing 2-10 ether functions, and mixtures thereof; preferably wherein the compound (ii) is selected from 2-hydroxypropyl methacrylate, 2-hydroxyethyl methacrylate and mixtures thereof.

10. An adhesive polyurethane composition according to claim 8 or claim 9, comprising from 0.05 to 1 meq/g, preferably from 0.06 to 0.5 meq/g, preferably from 0.08 to 0.4 meq/g, preferably from 0.1 to 0.3 meq/g, preferably from 0.12 to 0.25 meq/g, preferably from 0.15 to 0.2 meq/g of the functional group that is curable by free-radical polymerisation.

11. An adhesive polyurethane composition according to any of claims 1 to 11, wherein the cross-linking component (B) is obtained by reacting the polyisocyanate component (i) with the compound (iii) and wherein the compound (ii) is absent;
preferably wherein the degree of substitution of the polyisocyanate component (i) by the compound (iii) is in the range from 0.15 to 0.65, or in the range from 0.2 to 0.6, or in the range from 0.25 to 0.6, or in the range from 0.3 to 0.6.

12. An adhesive polyurethane composition according to any of claims 8 to 11, wherein the at least one compound (iii) comprises or consists of one or more C₁-C₃₀ aliphatic alcohols, preferably linear, branched or cyclic C₁-C₁₈ aliphatic alcohols, preferably linear, branched or cyclic C₁-C₁₂ aliphatic alcohols, preferably branched C₃-C₁₂ aliphatic alcohols, or branched C₆-C₁₈ aliphatic alcohols.

13. An adhesive polyurethane composition according to any preceding claim, wherein the molar ratio of unsubstituted isocyanate functions in the cross-linking component (B) to nucleophilic groups containing an active hydrogen atom in the polymer component (A) is at least 0.81, or at least 0.82, or at least 0.83, or at least 0.84, or at least 0.85, or at least 0.86, or at least 0.87, or at least 0.88, or at least 0.89, or at least 0.90, or at least 0.91, or at least 0.92, or at least 0.93, or at least 0.94, or at least 0.95.

14. An adhesive polyurethane composition according to any preceding claim, further comprising a photoinitiator, preferably from 0.05 to 5 wt% of a photoinitiator, preferably from 0.1 to 5 wt% of a photoinitiator, preferably from 0.2 to 2 wt% of a photoinitiator.

15. A method of preparing an adhesive polyurethane composition comprising:
(a) a first step of reacting:
(i) a polyisocyanate component having an average of 1.8 to 6 isocyanate functions per molecule;
with at least one of:
(ii) at least one compound comprising a functional group that is curable by free-radical polymerisation and further comprising a nucleophilic functional group containing an active hydrogen atom; and
(iii) at least one compound comprising a nucleophilic functional group containing an active hydrogen atom and that does not include a functional group that is curable by free-radical polymerisation;
wherein the total degree of substitution of the polyisocyanate component (i) by the compounds (ii) and (iii) is in the range from 0.1 to 0.7; to form a cross-linking component (B);
(b) a second step of combining cross-linking component (B) formed in step (a) with a polymer component (A) having a weight average molecular weight in the range of 1,000 to 100,000 Dalton, and containing an average per molecule of X nucleophilic functional groups containing an active hydrogen atom, wherein X represents a number having a value of at least 2,
wherein the amounts of the cross-linking component (B) and the polyol component (A) are selected such that molar ratio of unsubstituted isocyanate functions in the cross-linking component (B) to nucleophilic functional groups containing an active hydrogen atom in the polymer component (A) is at least 0.8.

## Patentansprüche

1. Klebstoffzusammensetzung, umfassend das Reaktionsprodukt aus Folgendem:
(A) einer Polymerkomponente, die ein gewichtsmittleres Molekulargewicht im Bereich von 1.000 bis 100.000 Dalton aufweist, und die einen Mittelwert je Molekül von X nukleophilen funktionellen Gruppen, die ein aktives Wasserstoffatom enthalten, enthält, wobei X eine Zahl darstellt, die einen Wert von mindestens 2 aufweist; und
(B) einer Vernetzungskomponente, erhalten durch Reagieren Folgendes:
(i) einer Polyisocyanat-Komponente, die einen Mittelwert von 1,8 bis 6 Isocyanat-Funktionen je Molekül aufweist;
mit mindestens einem aus Folgendem:
(ii) mindestens einer Verbindung, die eine funktionelle Gruppe umfasst, die durch radikalische Polymerisation härtbar ist, und weiter eine nukleophile funktionelle Gruppe umfasst, die ein aktives Wasserstoffatom enthält; und
(iii) mindestens einer Verbindung, die eine nukleophile funktionelle Gruppe umfasst, die ein aktives Wasserstoffatom enthält, und die keine funktionelle Gruppe einschließt, die durch radikalische Polymerisation härtbar ist;
wobei der Gesamtsubstitutionsgrad der Polyisocyanat-Komponente (i) durch die Verbindungen (ii) und (iii) im Bereich von 0,1 bis 0,7 liegt; und wobei das Molverhältnis von nicht substituierten Isocyanat-Funktionen in der Vernetzungskomponente (B) zu nukleophilen funktionellen Gruppen, die ein aktives Wasserstoffatom enthalten, in der Polymerkomponente (A) mindestens 0,8 beträgt.

2. Klebstoffzusammensetzung nach Anspruch 1, wobei X einen Wert von mindestens 2,2 oder mindestens 2,4 oder mindestens 2,6 oder mindestens 2,8 oder mindestens 3 oder mindestens 3,2 aufweist.

3. Klebstoffzusammensetzung nach Anspruch 1 oder Anspruch 2, wobei die Polymerkomponente ein Polyol, vorzugsweise ein Polyol ist, das einen Mittelwert von 2,8 bis 5 Hydroxygruppen je Molekül umfasst.

4. Klebstoffzusammensetzung nach Anspruch 3, wobei mindestens 50 Mol-% der Hydroxygruppen im Polyol in Polyolmolekülen gefunden werden, die 3 bis 5 Hydroxygruppen umfassen, oder wobei mindestens 60 Mol-% der Hydroxygruppen im Polyol in Polyolmolekülen gefunden werden, die 3 bis 5 Hydroxygruppen umfassen, oder wobei mindestens 70 Mol-% der Hydroxygruppen im Polyol in Polyolmolekülen gefunden werden, die 3 bis 5 Hydroxygruppen umfassen, oder wobei mindestens 80 Mol-% der Hydroxygruppen im Polyol in Polyolmolekülen gefunden werden, die 3 bis 5 Hydroxygruppen umfassen, oder wobei mindestens 90 Mol-% der Hydroxygruppen im Polyol in Polyolmolekülen gefunden werden, die 3 bis 5 Hydroxygruppen umfassen.

5. Polyurethan-Klebstoffzusammensetzung nach einem vorstehenden Anspruch, wobei die Polymerkomponente (A) ein Polyol ist, das aus hydroxy-terminierten Polyethern und hydroxy-terminierten Polyestern ausgewählt ist;
wobei der hydroxy-terminierte Polyether vorzugsweise ein alkoxyliertes Derivat einer Verbindung, die 3 bis 5 Hydroxygruppen umfasst, oder Gemischen davon ist;
wobei die Verbindung, die 3 bis 5 Hydroxygruppen umfasst, vorzugsweise aus Glycerin, Trimethylolpropan, Erythrit, Pentaerythrit, Pentan-1,2,4,5-tetrol, Dextrose und Gemischen davon ausgewählt ist, wobei die alkoxylierten Derivate vorzugsweise ethoxylierte Derivate, propoxylierte Derivate oder ethoxylierte-co-propoxylierte Derivate sind.

6. Polyurethan-Klebstoffzusammensetzung nach einem vorstehenden Anspruch, wobei die Polymerkomponente ein gewichtsmittleres Molekulargewicht im Bereich von 1.000 bis 50.000 Dalton, vorzugsweise im Bereich von 1.000 bis 20.000 Dalton, vorzugsweise im Bereich von 1.500 bis 10.000 Dalton aufweist;
wobei die Polymerkomponente vorzugsweise ein Äquivalentgewicht je nukleophiler funktioneller Gruppe von 200 bis 5.000, vorzugsweise von 500 bis 2.500, vorzugsweise von 1.000 bis 2.000 aufweist.

7. Polyurethan-Klebstoffzusammensetzung nach einem vorstehenden Anspruch, wobei die Polyisocyanat-Komponente (i) einen Mittelwert von 2 bis 4 Isocyanat-Funktionen je Molekül, vorzugsweise 2,1 bis 3,6 Isocyanat-Funktionen je Molekül, vorzugsweise 2,1 bis 3,5 Isocyanat-Funktionen je Molekül, vorzugsweise 2,2 bis 3,4 Isocyanat-Funktionen je Molekül, vorzugsweise 2,5 bis 3,4 Isocyanat-Funktionen je Molekül umfasst;
wobei die Polyisocyanat-Komponente vorzugsweise (i) ein trimerisiertes Diisocyanat der Formel D(R-NCO)₃ umfasst, wobei D eine Ringstruktur, ausgewählt aus Isocyanurat und Iminooxadiazindion, oder eine verzweigte Struktur, ausgewählt aus Biuret und Allophanat und Gemischen davon darstellt, und jedes R unabhängig eine geradkettige, verzweigte oder cyclische Alkylengruppe darstellt, die 2 bis 15 Kohlenstoffatome aufweist, oder eine Arylgruppe, die 6 bis 20 Kohlenstoffatome aufweist, darstellt, wobei das trimerisierte Diisocyanat trimerisiertes Hexamethylendiisocyanat ist.

8. Klebstoffzusammensetzung nach einem vorstehenden Anspruch, wobei die Vernetzungskomponente (B) durch Reagieren der Polyisocyanat-Komponente (i) mit der Verbindung (ii) und optional mit der Verbindung (iii) erhalten wird;
wobei der Substitutionsgrad der Polyisocyanat-Komponente vorzugsweise (i) durch die Verbindung (ii) im Bereich von 0,05 bis 0,5; oder von 0,05 bis 0,45 oder von 0,05 bis 0,4 oder von 0,8 bis 0,35 oder von 0,8 bis 0,3 oder von 0,1 bis 0,25 oder von 0,1 bis 0,2 oder von 0,12 bis 0,18 liegt;
vorzugsweise wobei der Gesamtsubstitutionsgrad der Polyisocyanat-Komponente (i) durch die Verbindungen (ii) und (iii) im Bereich von 0,15 bis 0,65 oder im Bereich von 0,2 bis 0,6 oder im Bereich von 0,25 bis 0,6 oder im Bereich von 0,3 bis 0,6 liegt.

9. Polyurethan-Klebstoffzusammensetzung nach Anspruch 8, wobei die mindestens eine Verbindung (ii) einen Olefinrest als die funktionelle Gruppe, die durch radikalische Polymerisation härtbar ist, umfasst; bevorzugt wobei die mindestens eine Verbindung (ii) aus hydroxy-substituierten Acrylatestern, hydroxy-substituierten Methacrylatestern und Gemischen davon ausgewählt ist; wobei die mindestens eine Verbindung (ii) vorzugsweise aus hydroxy-(C₂-C₂₀)alkyl-substituierten Methacrylatestern, polyalkoxylierten Monomethacrylatestern, die 2-10 Etherfunktionen enthalten, und Gemischen davon ausgewählt ist; vorzugsweise wobei die Verbindung (ii) aus 2-Hydroxypropylmethacrylat, 2-Hydroxyethylmethacrylat und Gemischen davon ausgewählt ist.

10. Polyurethan-Klebstoffzusammensetzung nach Anspruch 8 oder Anspruch 9, umfassend von 0,05 bis 1 meq/g, vorzugsweise von 0,06 bis 0,5 meq/g, vorzugsweise von 0,08 bis 0,4 meq/g, vorzugsweise von 0,1 bis 0,3 meq/g, vorzugsweise von 0,12 bis 0,25 meq/g, vorzugsweise von 0,15 bis 0,2 meq/g der funktionellen Gruppe, die durch radikalische Polymerisation härtbar ist.

11. Polyurethan-Klebstoffzusammensetzung nach einem der Ansprüche 1 bis 11, wobei die Vernetzungskomponente (B) durch Reagieren der Polyisocyanat-Komponente (i) mit der Verbindung (iii) erhalten wird, und wobei die Verbindung (ii) nicht vorhanden ist; wobei der Substitutionsgrad der Polyisocyanat-Komponente (i) durch die Verbindung (iii) vorzugsweise im Bereich von 0,15 bis 0,65 oder im Bereich von 0,2 bis 0,6 oder im Bereich von 0,25 bis 0,6 oder im Bereich von 0,3 bis 0,6 liegt.

12. Polyurethan-Klebstoffzusammensetzung nach einem der Ansprüche 8 bis 11, wobei die mindestens eine Verbindung (iii) einen oder mehrere aliphatische C₁-C₃₀ - Alkohole, vorzugsweise lineare, verzweigte oder cyclische aliphatische C₁-C₁₈-Alkohole, vorzugsweise lineare, verzweigte oder cyclische aliphatische C₁-C₁₂-Alkohole, vorzugsweise verzweigte aliphatische C₃-C₁₂-Alkohole oder verzweigte aliphatische C₆-C₁₈-Alkohole umfasst oder daraus besteht.

13. Polyurethan-Klebstoffzusammensetzung nach einem vorstehenden Anspruch, wobei das Molverhältnis von nicht substituierten Isocyanat-Funktionen in der Vernetzungskomponente (B) zu nukleophilen Gruppen, die ein aktives Wasserstoffatom enthalten, in der Polymerkomponente (A) mindestens 0,81 oder mindestens 0,82 oder mindestens 0,83 oder mindestens 0,84 oder mindestens 0,85 oder mindestens 0,86 oder mindestens 0,87 oder mindestens 0,88 oder mindestens 0,89 oder mindestens 0,90 oder mindestens 0,91 oder mindestens 0,92 oder mindestens 0,93 oder mindestens 0,94 oder mindestens 0,95 beträgt.

14. Polyurethan-Klebstoffzusammensetzung nach einem vorstehenden Anspruch, weiter umfassend einen Photoinitiator, vorzugsweise von 0,05 bis 5 Gew.-% eines Photoinitiators, vorzugsweise von 0,1 bis 5 Gew.-% eines Photoinitiators, vorzugsweise von 0,2 bis 2 Gew.-% eines Photoinitiators.

15. Verfahren zur Herstellung einer Polyurethan-Klebstoffzusammensetzung, umfassend:
(a) einen ersten Schritt zum Reagieren Folgendes:
(i) einer Polyisocyanat-Komponente, die einen Mittelwert von 1,8 bis 6 Isocyanat-Funktionen je Molekül aufweist;
mit mindestens einem aus Folgendem:
(ii) mindestens einer Verbindung, die eine funktionelle Gruppe umfasst, die durch radikalische Polymerisation härtbar ist, und weiter eine nukleophile funktionelle Gruppe umfasst, die ein aktives Wasserstoffatom enthält; und
(iii) mindestens einer Verbindung, die eine nukleophile funktionelle Gruppe umfasst, die ein aktives Wasserstoffatom enthält, und die keine funktionelle Gruppe einschließt, die durch radikalische Polymerisation härtbar ist;
wobei der Gesamtsubstitutionsgrad der Polyisocyanat-Komponente (i) durch die Verbindungen (ii) und (iii) im Bereich von 0,1 bis 0,7 liegt; um eine Vernetzungskomponente (B) zu bilden;
(b) einen zweiten Schritt zum Kombinieren der in Schritt (a) gebildeten Vernetzungskomponente (B) mit einer Polymerkomponente (A), die ein gewichtsmittleres Molekulargewicht im Bereich von 1.000 bis 100.000 Dalton aufweist, und einen Mittelwert je Molekül von X nukleophilen funktionellen Gruppen enthält, die ein aktives Wasserstoffatom enthalten, wobei X eine Zahl darstellt, die einen Wert von mindestens 2 aufweist,
wobei die Mengen der Vernetzungskomponente (B) und der Polyol-Komponente (A) ausgewählt sind, sodass das Molverhältnis von nicht substituierten Isocyanat-Funktionen in der Vernetzungskomponente (B) zu nukleophilen funktionellen Gruppen, die ein aktives Wasserstoffatom enthalten, in der Polymerkomponente (A) mindestens 0,8 beträgt.

## Revendications

1. Composition adhésive comprenant le produit de réaction de :
(A) un composant polymère présentant un poids moléculaire moyen en poids dans la plage de 1 000 à 100 000 Dalton, et contenant une moyenne par molécule de X groupes fonctionnels nucléophiles contenant un atome d'hydrogène actif, dans laquelle X représente un nombre présentant une valeur d'au moins 2 ; et
(B) un composant de réticulation obtenu par mise en réaction de :
(i) un composant polyisocyanate présentant une moyenne de 1,8 à 6 fonctions isocyanate par molécule ;
avec au moins un des éléments suivants :
(ii) au moins un composé comprenant un groupe fonctionnel qui est durcissable par polymérisation radicalaire et comprenant en outre un groupe fonctionnel nucléophile contenant un atome d'hydrogène actif ; et
(iii) au moins un composé comprenant un groupe fonctionnel nucléophile contenant un atome d'hydrogène actif et qui n'inclut pas de groupe fonctionnel qui est durcissable par polymérisation radicalaire ;
dans laquelle le degré total de substitution du composant polyisocyanate (i) par les composés (ii) et (iii) est dans la plage allant de 0,1 à 0,7 ; et dans laquelle le rapport molaire des fonctions isocyanate non substituées dans le composant de réticulation (B) sur les groupes fonctionnels nucléophiles contenant un atome d'hydrogène actif dans le composant polymère (A) est d'au moins 0,8.

2. Composition adhésive selon la revendication 1, dans laquelle X présente une valeur d'au moins 2,2, ou d'au moins 2,4, ou d'au moins 2,6, ou d'au moins 2,8, ou d'au moins 3, ou d'au moins 3,2.

3. Composition adhésive selon la revendication 1 ou la revendication 2, dans laquelle le composant polymère est un polyol, de préférence un polyol comprenant une moyenne de 2,8 à 5 groupes hydroxyle par molécule.

4. Composition adhésive selon la revendication 3, dans laquelle au moins 50 % mol des groupes hydroxyle dans le polyol se trouvent dans des molécules de polyol comprenant de 3 à 5 groupes hydroxyle, ou dans laquelle au moins 60 % mol des groupes hydroxyle dans le polyol se trouvent dans des molécules de polyol comprenant de 3 à 5 groupes hydroxyle, ou dans laquelle au moins 70 % mol des groupes hydroxyle dans le polyol se trouvent dans des molécules de polyol comprenant de 3 à 5 groupes hydroxyle, ou dans laquelle au moins 80 % mol des groupes hydroxyle dans le polyol se trouvent dans des molécules de polyol comprenant de 3 à 5 groupes hydroxyle, ou dans laquelle au moins 90 % mol des groupes hydroxyle dans le polyol se trouvent dans des molécules de polyol comprenant de 3 à 5 groupes hydroxyle.

5. Composition adhésive de polyuréthane selon l'une quelconque des revendications précédentes, dans laquelle le composant polymère (A) est un polyol sélectionné parmi les polyéthers à terminaison hydroxyle et les polyesters à terminaison hydroxyle ;
de préférence dans laquelle le polyéther à terminaison hydroxyle est un dérivé alkoxylé d'un composé comprenant de 3 à 5 groupes hydroxyle, ou des mélanges de ceux-ci ;
de préférence dans laquelle le composé comprenant de 3 à 5 groupes hydroxyle est sélectionné parmi le glycérol, le triméthylolpropane, l'érythritol, le pentaérythritol, le pentane-1,2,4,5-tétrol, le dextrose et des mélanges de ceux-ci, de préférence dans laquelle les dérivés alkoxylés sont des dérivés éthoxylés, des dérivés propoxylés, ou des dérivés éthoxylés-co-propoxylés.

6. Composition adhésive de polyuréthane selon l'une quelconque des revendications précédentes, dans laquelle le composant polymère présente un poids moléculaire moyen en poids dans la plage de 1 000 à 50 000 Dalton, de préférence dans la plage de 1 000 à 20 000 Dalton, de préférence dans la plage de 1 500 à 10 000 Dalton ;
de préférence dans laquelle le composant polymère présente un poids équivalent par groupe fonctionnel nucléophile de 200 à 5 000, de préférence de 500 à 2 500, de préférence de 1 000 à 2 000.

7. Composition adhésive de polyuréthane selon l'une quelconque des revendications précédentes, dans laquelle le composant polyisocyanate (i) comprend une moyenne de 2 à 4 fonctions isocyanate par molécule, de préférence 2,1 à 3,6 fonctions isocyanate par molécule, de préférence 2,1 à 3,5 fonctions isocyanate par molécule, de préférence 2,2 à 3,4 fonctions isocyanate par molécule, de préférence 2,5 à 3,4 fonctions isocyanate par molécule ;
de préférence dans laquelle le composant polyisocyanate (i) comprend un diisocyanate trimérisé de la formule D(R-NCO)₃, dans laquelle D représente une structure cyclique sélectionnée parmi l'isocyanurate et l'iminooxadiazindione ou une structure ramifiée sélectionnée parmi le biuret et l'allophanate et des mélanges de ceux-ci, et chaque R représente indépendamment un groupe alkylène linéaire, ramifié ou cyclique présentant de 2 à 15 atomes de carbone ou un groupe aryle présentant de 6 à 20 atomes de carbone, de préférence dans laquelle le diisocyanate trimérisé est du diisocyanate d'hexaméthylène trimérisé.

8. Composition adhésive selon l'une quelconque des revendications précédentes, dans laquelle le composant de réticulation (B) est obtenu par mise en réaction du composant polyisocyanate (i) avec le composé (ii) et facultativement avec le composé (iii) ;
de préférence dans laquelle le degré de substitution du composant polyisocyanate (i) par le composé (ii) est dans la plage allant de 0,05 à 0,5 ; ou de 0,05 à 0,45, ou de 0,05 à 0,4, ou de 0,8 à 0,35, ou de 0,8 à 0,3, ou de 0,1 à 0,25, ou de 0,1 à 0,2, ou de 0,12 à 0,18 ;
de préférence dans laquelle le degré total de substitution du composant polyisocyanate (i) par les composés (ii) et (iii) est dans la plage allant de 0,15 à 0,65, ou dans la plage allant de 0,2 à 0,6 ou dans la plage allant de 0,25 à 0,6, ou dans la plage allant de 0,3 à 0,6.

9. Composition adhésive de polyuréthane selon la revendication 8, dans laquelle l'au moins un composé (ii) comprend un motif oléfinique en tant que groupe fonctionnel qui est durcissable par polymérisation radicalaire ; de préférence dans laquelle l'au moins un composé (ii) est sélectionné parmi des esters acrylates substitués par hydroxy, des esters méthacrylates substitués par hydroxy, et des mélanges de ceux-ci ; de préférence dans laquelle l'au moins un composé (ii) est sélectionné parmi des esters méthacrylates substitués par hydroxy-(C₂-C₂₀)alkyle, des esters monométhacrylates polyalkoxylés contenant de 2 à 10 fonctions éther, et des mélanges de ceux-ci ; de préférence dans laquelle le composé (ii) est sélectionné parmi le méthacrylate de 2-hydroxypropyle, le méthacrylate de 2-hydroxyéthyle et des mélanges de ceux-ci.

10. Composition adhésive de polyuréthane selon la revendication 8 ou la revendication 9, comprenant de 0,05 à 1 meq/g, de préférence de 0,06 à 0,5 meq/g, de préférence de 0,08 à 0,4 meq/g, de préférence de 0,1 à 0,3 meq/g, de préférence de 0,12 à 0,25 meq/g, de préférence de 0,15 à 0,2 meq/g du groupe fonctionnel qui est durcissable par polymérisation radicalaire.

11. Composition adhésive de polyuréthane selon l'une quelconque des revendications 1 à 11, dans laquelle le composant de réticulation (B) est obtenu par mise en réaction du composant polyisocyanate (i) avec le composé (iii) et dans laquelle le composé (ii) est absent ;
de préférence dans laquelle le degré de substitution du composant polyisocyanate (i) par le composé (iii) est dans la plage allant de 0,15 à 0,65, ou dans la plage allant de 0,2 à 0,6, ou dans la plage allant de 0,25 à 0,6, ou dans la plage allant de 0,3 à 0,6.

12. Composition adhésive de polyuréthane selon l'une quelconque des revendications 8 à 11, dans laquelle l'au moins un composé (iii) comprend ou consiste en un ou plusieurs alcools aliphatiques en C₁-C₃₀, de préférence des alcools aliphatiques linéaires, ramifiés ou cycliques en C₁-C₁₈, de préférence des alcools aliphatiques linéaires, ramifiés ou cycliques en C₁-C₁₂, de préférence des alcools aliphatiques ramifiés en C₃-C₁₂, ou des alcools aliphatiques ramifiés en C₆-C₁₈.

13. Composition adhésive de polyuréthane selon l'une quelconque des revendications précédentes, dans laquelle le rapport molaire des fonctions isocyanate non substituées dans le composant de réticulation (B) sur les groupes nucléophiles contenant un atome d'hydrogène actif dans le composant polymère (A) est d'au moins 0,81, ou d'au moins 0,82, ou d'au moins 0,83, ou d'au moins 0,84, ou d'au moins 0,85, ou d'au moins 0,86, ou d'au moins 0,87, ou d'au moins 0,88, ou d'au moins 0,89, ou d'au moins 0,90, ou d'au moins 0,91, ou d'au moins 0,92, ou d'au moins 0,93, ou d'au moins 0,94, ou d'au moins 0,95.

14. Composition adhésive de polyuréthane selon l'une quelconque des revendications précédentes, comprenant en outre un photo-initiateur, de préférence de 0,05 à 5 % en poids d'un photo-initiateur, de préférence de 0,1 à 5 % en poids d'un photo-initiateur, de préférence de 0,2 à 2 % en poids d'un photo-initiateur.

15. Procédé de préparation d'une composition adhésive de polyuréthane comprenant :
(a) une première étape de mise en réaction de :
(i) un composant polyisocyanate présentant une moyenne de 1,8 à 6 fonctions isocyanate par molécule ;
avec au moins un des éléments suivants :
(ii) au moins un composé comprenant un groupe fonctionnel qui est durcissable par polymérisation radicalaire et comprenant en outre un groupe fonctionnel nucléophile contenant un atome d'hydrogène actif ; et
(iii) au moins un composé comprenant un groupe fonctionnel nucléophile contenant un atome d'hydrogène actif et qui n'inclut pas de groupe fonctionnel qui est durcissable par polymérisation radicalaire ;
dans lequel le degré total de substitution du composant polyisocyanate (i) par les composés (ii) et (iii) est dans la plage allant de 0,1 à 0,7 ; pour former un composant de réticulation (B) ;
(b) une deuxième étape de combinaison du composant de réticulation (B) formé à l'étape (a) avec un composant polymère (A) présentant un poids moléculaire moyen en poids dans la plage de 1 000 à 100 000 Dalton, et contenant une moyenne par molécule de X groupes fonctionnels nucléophiles contenant un atome d'hydrogène actif, dans lequel X représente un nombre présentant une valeur d'au moins 2,
dans lequel les quantités du composant de réticulation (B) et du composant polyol (A) sont sélectionnées de telle sorte que le rapport molaire des fonctions isocyanate non substituées dans le composant de réticulation (B) sur les groupes fonctionnels nucléophiles contenant un atome d'hydrogène actif dans le composant polymère (A) est d'au moins 0,8.
